# EUROPEAN PATENT SPECIFICATION

(11) **EP 2 906 301 B1**
(45) Date of publication and mention of the grant of the patent: **07.09.2022**
(21) Application number: 13776511.1
(22) Date of filing: 15.10.2013
(51) Int. Cl.: A61K 8/44, A61K 8/46, A61Q 17/04, A61K 8/86, A61K 8/02, A61Q 5/06, A61K 8/92, A61K 8/04

(54) **AQUEOUS WAX DISPERSIONS AND HAIR STYLING COMPOSITIONS CONTAINING THEM**
WÄSSRIGE WACHSDISPERSIONEN UND HAARSTYLING-ZUSAMMENSETZUNGEN DAMIT
DISPERSIONS AQUEUSES DE CIRE ET COMPOSITIONS DE COIFFAGE LES CONTENANT

(30) Priority: 15.10.2012 US 201213651732; 15.10.2012 US 201213651710; 15.10.2012 US 201213651751; 15.10.2012 US 201213651768
(43) Date of publication of application: 19.08.2015
(73) Proprietor: L'Oréal, 75008 Paris (FR)
(72) Inventor: PISTORIO, Bradford, Joseph, Clark, NJ 07066 (US); SIMONNET, Jean-Thierry, Mamaroneck, NY 10543 (US); SINGER, Jim, Mitchell, South Orange, NJ 07079 (US); SAMAIN, Henri, F-91570 Bievres (FR)
(74) Representative: Casalonga
(86) International application number: PCT/EP2013/071516
(87) International publication number: WO 2014/060405

(56) References cited:
- EP-A1- 1 269 974
- DE-A1- 10 150 726
- FR-A1- 2 844 190
- US-A1- 2010 278 770
- DATABASE GNPD [Online] MINTEL; 29 September 2007 (2007-09-29), anonymous: "Wax-in-Cream UV Cut (Arrange Make)", XP055678045, retrieved from www.gnpd.com Database accession no. 807411

## Description

### FIELD OF THE INVENTION

The present invention relates to aqueous wax dispersions and methods of using these dispersions on various substrates. More particularly, the invention is directed to an aqueous dispersion comprising a solid wax particle, a surfactant mixture comprising a nonionic surfactant and an ionic surfactant, and water, and to a hair styling composition comprising it.

### BACKGROUND OF THE INVENTION

Consumer products such as cosmetics, personal care, and household products, as well as pharmaceutical and industrial products, employ ingredients that allow these products to form a film or coating on various substrates such as keratinous substrates (e.g., hair and skin), hard surfaces (e.g., wood and metal), and other non-keratinous substrates, (e.g., fabrics and articles). Those ingredients which help form a film or coating on the surface of a substrate may be chosen from a variety of raw materials such as waxes, polymers, resins and oils. At the same time, products which employ these ingredients are designed to impart certain desirable properties such as shine, water resistance, transfer resistance, scratch resistance, color and a glazed appearance to a surface.

In particular, waxes are highly desirable in cosmetics and personal care products as well as in household/industrial products in order to provide properties such as shine, smoothness, and slipperiness to various types of surfaces, as well as a protective coating against external factors such as exposure to water or moisture and physical rubbing. In the area of cosmetics, hair styling products which contain one or more of the above-mentioned ingredients can be used to impart shape or style to the hair and/or to help maintain a particular hair style. Makeup cosmetic products which employ these ingredients are used to enhance the appearance of the skin, lips and eyelashes. For example, mascara products employ waxes and polymers which help shape or curl the eyelashes. Sunscreen products and other cosmetics can also use these ingredients to provide a water-resistant film or coating on the skin and hair, and also to help maintain the appearance and condition of skin and hair upon exposure to extreme environmental conditions, for example, high or low humidity. In addition, these ingredients can provide structure and texture to the products and a certain feel and texture to a substrate.

Nevertheless, consumers continuously seek new products with improved performance and therefore, challenges still exist today in terms of optimizing or enhancing the performance of these ingredients in various products.

For instance, with regard to hair styling/shaping products, the effects of temporary styling products generally last for only a relatively short period of time. Thus, in order to re-shape or re-style the hair, the consumer has to either reapply a hair styling product and/or wash the hair again followed by a new application of the product and restyling the hair. Permanent styling also has the drawback of having to treat the hair with permanent waving or straightening/relaxing chemical treatments which may damage the hair.

Moreover, the formulation of waxes, polymers, resins and oils in various galenic forms such as sprays, foams, emulsions, gels, mousses, pastes and sticks may pose a challenge since some of these ingredients may not be easily introduced and/or dispersed into these galenic forms. In addition, the final formulas using these ingredients have to remain stable over time.

For example, waxes are traditionally employed in a paste or pomade but may not be easily formulated in a spray or foam product, particularly at a concentration that will be sufficient to impart the desirable attributes obtained from a wax ingredient. The type of wax may also affect the stability and dispersion of the wax particles in the formulation since wax particles could agglomerate. Certain waxes may also result in an undesirable rough texture and/or sticky and tacky feel of the product and/or to the treated substrate. In paste formulas, waxes are first melted and then blended with oils, plasticizers, clays and/or any other additives. In other words, formulating with waxes still poses a challenge with respect to optimizing the benefits that can be obtained from the wax or waxes themselves. Thus, there still exists a need to improve how ingredients such as waxes, polymers, resins and oils can be formulated into various galenic forms, and at the same time, optimize the benefits derived from these ingredients and enhance the performance of other ingredients.

Thus, various technologies directed towards the use of waxes, polymers, resins and oils have been developed. For example, shape memory polymers (SMPs) have been found to have the ability to change shape and therefore, provide certain materials made of such polymers with the ability to change their shapes or revert back to their original shape upon deformation, particularly, when an external stimuli such as heat or light is applied; SMPs may be used in packaging films, fabrics and medical devices (Marc Biehl and Andreas Lendlein (2007). Shape Memory Polymers, Materials Today. 10 (4), pp. 20-28). In the area of cosmetics and hair care, US20080311050 and US20070275020 teach the use of shape memory polymers in hair treatment compositions. However, SMPs are typically complex polymer systems which may pose challenges in synthesis procedures and formulation in terms of the choice of solvents and delivery/galenic form.

Other teachings, such as DE2810130, disclose applying a polyamide powder onto hair and heating the hair to bond the hair in a particular style; however, this reference does not teach that the hair can be re-styled or re-positioned and appears to be directed to wigs. WO8904653 and WO8901771 disclose the use of heat-activated hair styling compositions containing water-soluble polyethylene oxide polymers. EP1174113, US7998465 and US20120070391 are directed to the use of specific polymers, including thermofusible polymers, heat-expandable particles comprising certain polymers, and polysiloxanes and silanes. However, the use of polymers may still result in sticky formulas, may be difficult to formulate into a stable dispersion as a result of compatibility issues with surfactants, and do not necessarily provide a long lasting coat or film or the ability to easily re-style or re-position the hair without reapplying a product, for example.

US7871600, US6066316, JP2003012478, US20060292095 and US20060263438 teach the preparation of wax and oil dispersions in hair cosmetic compositions. For instance, US7871600 teaches the use of a wax dispersion in a hair styling composition. However, said composition additionally requires a styling polymer and a relatively high amount of wax of from 30% to 45% by weight of the composition. US6066316 discloses fine wax dispersions containing wax, an amphoteric surfactant and a nonionic surfactant where the size of the wax particles is about 30 nm and the nonionic surfactant is directed towards a specific class, i.e., polyoxypropylene alkyl ethers. JP2003012478 teaches a hair composition with hair-remodelling properties comprising an oil soluble material, a nonionic surfactant and water; the oil soluble material contains fatty acid, higher alcohol and wax. US20060292095 and US20060263438 disclose dispersions of oil particles calibrated to specific sizes and shapes; these particles are for use in sunscreen and skin care compositions. Nevertheless, the preparation of wax and oil particle dispersions and formulating with these dispersions in various galenic forms may still pose challenges, particularly since there are a number of factors to consider when working with wax and oil particles such as size, shape, hardness and melting point. Another consideration is the challenge of finding a convenient and easy way of optimizing the benefits that are delivered to substrates treated with such dispersions and compositions containing these dispersions.

Thus, it is an object of the present invention to provide a material comprising a wax, that is, a wax dispersion comprising wax particles having certain physical properties, wherein the wax dispersion can be employed in various galenic forms. It is also an object of the present invention to provide a novel way of imparting certain desirable properties to the surface of a substrate using said wax dispersion and/or compositions containing the wax dispersion.

Another subject of the present invention is a hair styling composition comprising an aqueous dispersion of the invention, in order to help maintaining the shape of hair or to re-position/re-style the hair without reapplication of product, to provide humidity resistance and impart other desirable properties to hair such as shine, conditioning, softness and combability as well while having good aesthetic features.

### BRIEF SUMMARY OF THE INVENTION

The present invention relates to a composition comprising:
- an aqueous dispersion containing:
   (a) at least one solid wax particle having a particle size ranging from equal to or greater than 1 micron to 25 microns and comprising at least one wax having a melting point of greater than 35°C;
   (b) a surfactant mixture comprising:
      (i) at least one nonionic surfactant as defined in claim 1; and
      (ii) at least one ionic surfactant comprising at least one specific anionic surfactant as defined in claim 1; and
   (c) water
- and a carrier preferably being a cosmetically acceptable carrier chosen from water, volatile organic solvents, non-volatile organic solvents, silicones, polyols, glycols, glycol ethers, oils, and mixtures thereof, wherein the at least one wax is present in an amount of from 1% to 5% by weight based on the total weight of the composition.

Furthermore, the present invention relates to composition which is a hair styling composition

The present invention relates also to a method of coating hair, the method comprising:
(a) applying onto the hair, a composition as defined in the present disclosure, and
(b) heating the hair in order to melt the at least one solid wax particle.

### Brief Description of the Figures

FIG. 1 shows optical microscopy views of wax dispersions comprising solid particles of beeswax having particle sizes ranging from 8-12 microns, 2-8 microns, 1-6 microns and 2-10 microns.

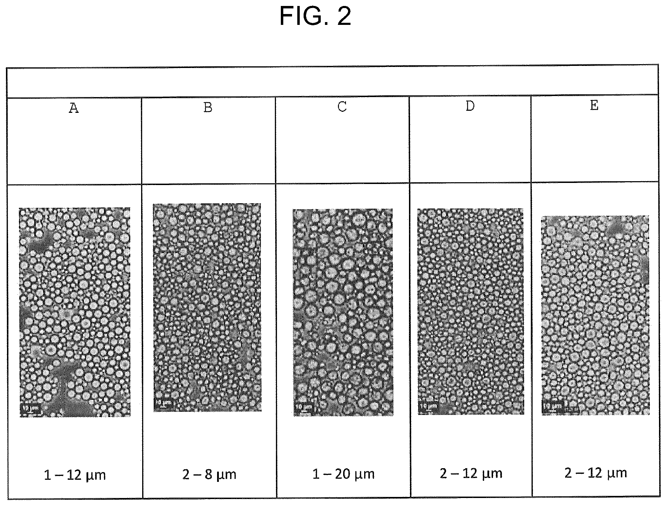

FIG. 2 shows optical microscopy views of wax dispersions comprising solid particles of beeswax having particle sizes ranging from 1-12 microns, 2-8 microns, 1-20 microns and 2-12 microns.

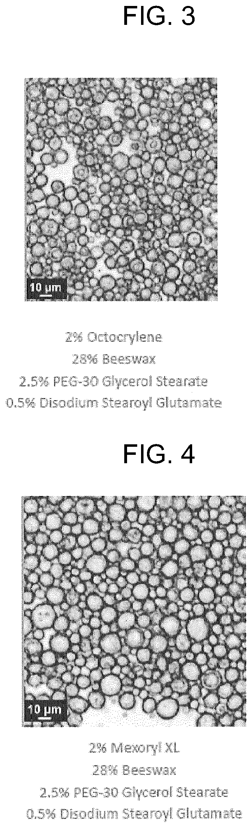

FIG. 3 and FIG. 4 show optical microscopy views of wax dispersions comprising solid particles and sunscreen agents.

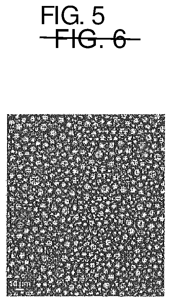

FIG. 5 optical microscopy view of a hair styling composition containing a wax dispersion comprising solid particles of beeswax having a particle size ranging from 4 - 10 microns.

### DETAILED DESCRIPTION OF THE INVENTION

As used herein, the expression "at least one" means one or more and thus includes individual components as well as mixtures/combinations.

The term "comprising" (and its grammatical variations) as used herein is used in the inclusive sense of "having" or "including" and not in the exclusive sense of "consisting only of". The terms "a" and "the" as used herein are understood to encompass the plural as well as the singular.

"Wax" as used herein means a hydrocarbon material, natural or synthetic, and having a melting point in the ranges disclosed below. Polymers and copolymers are included in this definition. Wax as used herein may also include a material composed of several components, including wax esters such as those derived from carboxylic acids and fatty alcohols, wax alcohols, and hydrocarbons.

"Film former" or "film forming agent" as used herein means a polymer or resin that leaves a film on the hair to which it is applied, for example, after a solvent accompanying the film former has evaporated, absorbed into and/or dissipated on the hair.

"Substituted" as used herein, means comprising at least one substituent. Non-limiting examples of substituents include atoms, such as oxygen atoms and nitrogen atoms, as well as functional groups, such as acyloxyalky groups, carboxylic acid groups, amine or amino groups, acylamino groups, amide groups, halogen containing groups, ester groups, thiol groups, sulphonate groups, thiosulphate groups, siloxane groups, and polysiloxane groups. The substituent(s) may be further substituted.

As used herein, the phrase "salts and derivatives thereof" is intended to mean all salts and derivatives comprising the same functional structure as the compound they are referring to, and that have similar properties.

As used herein, the term "applying a composition onto hair" and variations of this phrase are intended to mean contacting the hair, with at least one of the compositions of the invention, in any manner.

As used herein, "formed from," means obtained from chemical reaction of, wherein "chemical reaction," includes spontaneous chemical reactions and induced chemical reactions. As used herein, the phrase "formed from," is open ended and does not limit the components of the composition to those listed.

The term "stable" as used herein means that the composition does not exhibit phase separation and/or crystallization.

The term "treat" (and its grammatical variations) as used herein refers to the application of the aqueous dispersion and compositions containing the dispersion onto the surface of a substrate.

The term "shaping" (and its grammatical variations) as used herein includes styling or placing
hair, in a particular arrangement, form or configuration; or altering the curvature of hair; or re-positioning
hair to a different arrangement, form or configuration.

The compositions and methods of the present invention can comprise, consist of, or consist essentially of the essential elements and limitations of the invention described herein, as well as any additional or optional ingredients, components, or limitations described herein or otherwise useful.

It was surprisingly and unexpectedly discovered that the solid wax particles of the aqueous dispersion of the present disclosure can be prepared in a controlled manner by using a surfactant mixture that employs a combination of a nonionic surfactant and an ionic surfactant and following an emulsification process. As a result, a fine dispersion of micron-sized wax particles of a narrow particle size distribution and with minimal coalescence or agglomeration can be obtained. Moreover, the solid wax particles in the aqueous dispersion of the present disclosure are advantageously substantially homogeneous with respect to their shape.

Furthermore, the aqueous dispersion of the present disclosure is formulated into compositions of various galenic forms such as gels, mousses, lotions, creams, pastes, ointments, sprays and foams. It was found that when the aqueous dispersion of the present disclosure was added into one of these galenic forms, the solid wax particles remained homogeneously and finely dispersed in the composition and said composition is stable even during storage and exhibits no agglomeration or precipitation of the solid wax particles. Moreover, the resulting composition exhibits reduced or minimized stickiness or tackiness that is generally attributed to the use of waxes.

The compositions containing the aqueous dispersion of the present disclosure can be applied onto hair to form a film or coating on the surface of hair.

It was also surprisingly and unexpectedly found that when the hair treated with said film or coating is exposed to heat, additional benefits to the hair are achieved such as better adhesion and re-shapeability. It was also found that the treated hair may undergo further re-shaping and re-positioning when it is re-heated without the need for reapplication of the composition containing the aqueous dispersion of the present disclosure. The compositions containing the aqueous dispersion of the present disclosure also impart a clean and natural feel on the hair, despite the presence of wax.

Moreover, while the compositions containing the aqueous dispersion impart a coating or film onto hair, said compositions may easily be removed from the hair by washing with water or with conventional cleansing agents.

Although not wanting to be bound by any particular theory, it is believed that upon applying the aqueous dispersion onto hair in conjunction with heating the hair to a temperature around or above the melting point of the wax comprising the solid wax particle, the solid wax particles melt or soften, thereby allowing for the film or coating to be re-positioned on the hair and/or to adhere better to the hair.

### SOLID WAX PARTICLE

The at least one solid wax particle of the aqueous dispersion has a particle size ranging from equal to or greater than 1 micron to 25 microns, or such as from 2 microns to 25 microns, or such as from 3 microns to 25 microns.

Furthermore, the particle size of the at least one solid wax particle in the aqueous dispersion of the present disclosure may range from from microns to 25 microns, or such as from 5 microns to 12 microns, or such as from 5 microns to 10 microns.

The term "particle size" as used herein refers to the diameter of the particle. For non-spherical particles, the particle size refers to the largest diameter of the particles, i.e., the diameter in the dimension having the largest diameter.

Preferably, the solid wax particles in the aqueous dispersion of the present disclosure have a narrow particle size distribution, that is, the average difference in the particle sizes of the solid wax particles in an aqueous dispersion of the present disclosure is not more than 20 microns, or not more than 15 microns, or not more than 10 microns, or not more than 8 microns, or not more than 6 microns, or not more than 2 microns.

The shape of the solid wax particle may be spherical or ellipsoidal or oval. The terms "spherical" or ellipsoidal" or "oval" as used herein also mean that the solid wax particle has a uniform and substantially spherical or ellipsoidal or oval shape. The term "substantially" as used in the context of the shape of a spherical particle means that the particle is of substantially isotropic shape, i.e., it has a relatively regular morphology.

Thus, the ratio of the lengths of the longest to the shortest perpendicular axes of the particle cross section can be at 1:1 or at 1.5:1 or at 2:1 or at 3:1. Moreover, a line of symmetry is not required when the solid wax particle has a spherical shape. Further, the solid wax particle may have surface texturing, such as lines or indentations or protuberances that are small in scale when compared to the overall size of the solid wax particle and still be substantially spherical or ellipsoidal or oval.

The solid wax particles in the aqueous dispersion of the present disclosure are preferably substantially homogeneous with respect to their shape and particle size distribution. The term "substantially" as used in this context means that 50% or more of the solid wax particles in an aqueous dispersion of the present disclosure are of the same spherical, ellipsoidal or oval shape and of the same particle size.

The particle size, particle size distribution, and shape of the solid wax particle of the present disclosure may be evaluated by any known method such as those described in US patent application number 2006/0292095, for example, laser diffraction, ultrasonic extinction (acoustic spectroscopy), photo cross-correlation spectroscopy, granulometry, and image analysis (optical microscopy).

The solid wax particles of the present disclosure have a melting point greater than 35°C, such as from between greater than 35°C to 250°C, or such as from between greater than 35°C to 120°C, or such as from between 40°C to 100°C.

Moreover, the solid wax particles comprise at least one wax having a melting point greater than 35°C, such as from between greater than 35°C to 250°C or such as from between 40°C to 100°C. The at least one wax having a melting point greater than 35°C is defined as having a reversible change of solid/liquid state. The melting point of a wax in solid form is the same as the freezing point of its liquid form, and depends on such factors as the purity of the substance and the surrounding pressure. The melting point is the temperature at which a solid and its liquid are in equilibrium at any fixed pressure. A solid wax begins to soften at a temperature close to the melting point of the wax. With increasing temperature, the wax continues to soften/melt until at a particular temperature, the wax completely becomes liquid at a standard atmospheric pressure. It is at this stage that an actual melting point value is given for the material under consideration. When heat is removed, the liquefied wax material begins to solidify until the material is back in solid form. By bringing the wax material to the liquid state (melting), it is possible to make it miscible with other materials such as oils, and to form a microscopically homogeneous mixture. However, when the temperature of the mixture is brought to room temperature, recrystallization of the wax with the other materials in the mixture may be obtained.

The melting points of the wax(e)s and the solid wax particles of the aqueous dispersion of the present disclosure may be determined according to known methods or apparatus such as by differential scanning calorimetry, Banc Koffler device, melting point apparatus, and slip melting point measurements.

The wax(es) which comprises the at least one solid wax particle of the present disclosure and have a melting point of greater than 35°C is chosen from waxes that are solid or semisolid at room temperature.

The wax(es) which comprises the at least one solid wax particle of the present disclosure may be chosen from waxes that have hardness values ranging from 0.001 MPa (Mega Pa) to 15 MPa, or such as from 1 MPa to 12 MPa, or such as from 3 MPa to 10 MPa.

The hardness of the wax may be determined by any known method or apparatus such as by needle penetration or using the durometer or texturometer.

The wax comprising the at least one solid wax particle of the present disclosure is chosen from natural and synthetic waxes.

Natural waxes include animal, vegetable/plant, mineral, or petroleum derived waxes. They are typically esters of fatty acids and long chain alcohols. Wax esters are derived from a variety of carboxylic acids and a variety of fatty alcohols. The waxes comprising the solid wax particle of the present disclosure may also be known as solid lipids.

Examples of waxes comprising the at least one solid wax particle of the present disclosure include, but are not limited to, beeswax, hydrogentated alkyl olive esters (commercially available under the trade name phytowax olive), carnauba wax, candelilla wax, ouricoury wax, Japan wax, cork fibre wax or sugar cane wax, rice wax, montan wax, paraffin wax, lignite wax or microcrystalline wax, ceresin or ozokerite, palm kernel glycerides/hydrogenated palm glycerides and hydrogenated oils such as hydrogenated castor oil or jojoba oil, sugarcane, retamo, bayberry, rice bran, soy, castor, esparto, japan waxes, hydroxyoctacosanyl hydroxystearate, Chinese wax, cetyl palmitate, lanolin, shellac, and spermaceti; synthetic waxes such as those of the hydrocarbon type and polyethylene waxes obtained from the polymerization or copolymerization of ethylene, and Fischer-Tropsch^{®} waxes, or else esters of fatty acids, such as octacosanyl stearate, glycerides which are solid at temperatures of above 35°C, silicone waxes, such as alkyl- or alkoxydimethicones having an alkyl or alkoxy chain ranging from 10 to 45 carbon atoms, poly(di)methylsiloxane esters which are solid at 30°C and whose ester chain comprising at least 10 carbon atoms, or else di(1,1,1-trimethylolpropane) tetrastearate, which is sold or manufactured by Heterene under the name HEST^{®} 2T-4S, and mixtures thereof.

Other examples of waxes or solid lipids include C20-40 di- and triglycerides, including those which contain unsaturated fatty acids, C20-40 fatty alcohols, C2-40 fatty amines and their compounds, and sterols.

The table below lists waxes whose melting points are greater than 35°C and which are suitable for use in accordance with the present disclosure:

| INCI name and/or Trade name | Melting point (mp) |
|---|---|
| Paraffin wax | 57.3 °C |
| Stearic alcohol | 58.8 °C |
| Carnauba wax | 82.3 °C |
| Ozokerite | 66.8 °C |
| microcrystalline wax | 83.3 °C |
| polyethylene wax | 95.6 °C * |
| Hydrogenated Castor oil | 85.07 °C |
| synthetic beeswax | 51.2 °C * |
| wax AC 540 | 98.4 °C * |
| Beeswax | 62.6 °C |
| Candelilla wax | 64.3 °C |
| Hydroxyoctacosanyl Hydroxystearate | 76.8 °C |
| Hydrogenated Castor wax | 81.7 °C |
| wax AC 400 | 86.3 °C |
| PVP/Eicosene Copolymer | 37.8 °C |
| polyethylene wax | 83.9 °C |
| Hydrogenated Jojoba wax | 69.4 °C |
| palm butter | 58.4 °C |
| rice bran wax | 78.6 °C * |
| sumac wax | 48.3 °C |
| polyglycerol beeswax | 63.1 °C |
| Tricontanyl/PVP | 68.8 °C * |
| C20-40 Alkyl Stearate | 72.5 °C |
| siliconyl beeswax | 53.4 °C |
| Stearyl Stearate | 57.1 °C |
| polyethylene wax | 71.8 °C |
| polyethylene wax | 92.9 °C |
| ceresin wax | 60.1 °C |
| Ultrabee WD | 61.3 °C |
| Phytowax Olive 14 L 48 (hydrogenated myristyl olive esters) | 46.02 °C |
| Phytowax Olive 18 L 57 (hydrogenated stearyl olive esters) | 58.6 °C |
| Alcohol polyethylene wax | 95.7 °C |
| Koster wax K82P (anc.K80P) | 69.6 °C |
| Citrus Aurantium Dulcis (Orange) Peel Wax | 40.7 °C |
| Pentaerythritol Distearate | 48.5 °C |
| Theobroma Grandiflorum Seed Butter | 36.94 °C |
| DI 18/22 ADIPATE | 64.13 °C |
| DI 18/22 SEBACATE | 66.44 °C |
| DI 18/22 OCTANEDIOATE | 75.15 °C |
| Helianthus Annuus (Sunflower) Seed Wax | 75.46 °C |
| K82P-S | 67.97 °C |
| K82P-VS | 66.20 °C |
| Silicone resin wax (Dow Corning^{®} SW-8005) | 54.3 - 65.6°C |
| Polymethylalkyl dimethylsiloxane | 67.8 °C * |
| Alcohol polyethylene wax | 76.2 °C |
| Pentaerythrityl tetrastearate | 63.0 °C |
| Tetracontanyl Stearate | 65.1 °C |
| fatty acid wax | 63.7 °C |
| Fischer-tropsch wax | 79.3 °C * |
| behenyl alcohol | 66.9 °C |
| alkyl dimethicone wax | 57.0°C |
| Stearyl Benzoate | 40.6 °C |
| Berry wax | 47.5 °C |
| Chinese insect wax | 81.1 °C * |
| Shellac wax | 73.8 °C * |
| Behenyl fumarate | 74.5 °C |
| Koster BK-42 | 40.5 °C * |
| Koster KPC-56 | 58.5 °C |
| Koster KPC-60 | 61.7 °C |
| Koster KPC-63 | 65.2 °C |
| Koster KPC-80 | 55.6 °C |
| siliconyl candellila wax | 66.8 °C |
| Koster BK-37 | 38.0 °C |
| Ditrimethylolpropane tetrastearate | 46.5 °C |
| Synthetic Wax | 70.7 °C |
| Clariant Licowax KST 1 | 55.2 °C |
| Betawax RX-13750 | 72.0 °C |
| Dipentaerythrytol hexastearate | 67.7 °C |
| Ditrimethylolpropane tetrabehenate | 67.5 °C |
| Behenyl methacrylate grafted PDMS | 48.6 °C |
| Jojoba esters | 56.7 °C |
| Waxolive | 55.8 °C |
| Inholive | 40.3 °C |
| Phytowax Ricin 16 L 64 | 69.1 °C * |
| Phytowax Ricin 22 L 73 | 76.6 °C |
| Burco LB-02 | 45.1 °C |
| Hydrogenated Castor Oil Isostearate | 52.5 °C |
| Hydrogenated Castor Oil Isostearate | 54.0 °C * |
| Vegetable Wax | 81.0 °C |
| Hydrogenated Macadamia Seed Oil | 51.49 °C |
| Synthetic Wax | 51.4 °C |
| Dioctadecyl Carbonate | 56.7 °C |
| Montan Wax | 63.4 °C |
| Citrus Medica Limonum (Lemon) Peel Extract | 58.3 °C |

| | |
|---|---|
| *with several melting point peaks | |

Particularly preferred waxes having a melting point of greater than 35°C are beeswax, commercially available from various suppliers, hydrogenated stearyl olive ester, and commercially available from the supplier Sophim under the tradename, Phytowax Olive 18 L 57, hydrogenated myristyl olive ester, and commercially available from the supplier Sophim under the tradename, Phytowax Olive 14 L 48, VP/eicosene copolymer, commercially available from the supplier ISP under the tradenames, Antaron^{®} V 220 or Ganex^{®} V 220F, and ditrimethyloylpropane tetrastearate, commercially available from the supplier Heterene under the tradename, HEST 2T-4S.

Other particularly preferred waxes having a melting point of greater than 35°C are silicone waxes, including silsesquioxane resin waxes such as C30-45 alkyldimethylsilyl propylsilsesquioxane, commercially available as DOW CORNING SW-8005 C30 Resin Wax, from the company Dow Corning and such as those described in WO2005/100444.

The wax(es) which comprises the at least one solid wax particle of the present disclosure have a melting point of greater than 35°C, or may range from 40°C to 100°C, or such as from 40°C to 80°C. The wax(es) which comprises the at least one solid wax particle of the present disclosure may be chosen from soft waxes and from hard waxes. Soft waxes may be defined as those waxes which have a melting point of below 70°C, and preferably, a melting point of below 60°C. Hard waxes may be defined as those waxes which have a melting point of equal to or greater than 70°C, and preferably, a melting point of equal to or greater than 60°C.

According to one embodiment, soft waxes according to the present disclosure include, but are not limited to, Paraffin wax, stearic alcohol, ozokerite, synthetic beeswax, beeswax, candelilla wax, PVP/eicosene copolymer, hydrogenated jojoba wax, palm butter, sumac wax, polyglyceryl beeswax, tricontanyl/PVP, siliconyl beeswax, stearyl stearate, ceresin wax, hydrogenated myristyl olive esters (e.g., phytowax olive 14 L 48), hydrogenated stearyl olive esters (e.g., phytowax olive 18 L 57), Koster K82P, orange peel wax, Pentaerythritol distearate, Theobroma Grandiflorum Seed Butter, silicone resin wax, Polymethylalkyl dimethylsiloxane, Pentaerythrityl tetrastearate, Tetracontanyl Stearate, fatty acid wax, behenyl alcohol, alkyl dimethicone wax, Stearyl Benzoate, Berry wax, koster wax, siliconyl candelilla wax, Ditrimethylolpropane tetrastearate, Clariant Licowax KST 1, Dipentaerythrytol hexastearate, Ditrimethylolpropane tetrabehenate, Behenyl methacrylate gréffé PDMS, jojoba esters, waxolive, inholive, phytowax ricin 16 L 64, hydrogenated macadamia seed oil, synthetic wax, dooctadecyl carbonate, montan wax, lemon peel extract, ditrimethyloylpropane tetrastearate, and C30-45 alkyldimethylsilyl propylsilsesquioxane.

According to one embodiment, hard waxes according to the present disclosure, include, but are not limited to, carnauba wax, microcrystalline wax, polyethylene wax, hydrogenated castor oil, wax AC 540, Hydroxyoctacosanyl Hydroxystearate, hydrogenated castor wax, wax AC 400, rice bran wax, C20-40 alkyl stearate, Alcohol polyethylene wax, octanedioate, sunflower seed wax, fischer-tropsch wax, Chinese insect wax, shellac wax, benehyl fumarate, synthetic wax, betsawax RX-13750, phytowax ricin 22 L 73, and vegetable wax.

The wax having a melting point of greater than 35°C and comprising the at least one solid wax particle of the present disclosure may be employed in an amount ranging from 10% to 80% by weight, or preferably from 15% to 60% by weight, or preferably from 20% to 40% by weight, based on the total weight of the aqueous dispersion of the present disclosure, including all ranges and subranges therebetween.

In certain embodiments, the aqueous dispersions of the present disclosure comprise solid wax particles having different properties with respect to hardness and/or melting point and/or shape and/or size.

### ADDITIONAL INGREDIENTS

The solid wax particle can further comprise additional ingredients such as waxes having melting points of 35°C or less, oils, emulsifying polymers, silicas, talc, clays, ceramides, and perfumes. These additional ingredients can be added during the time of making the aqueous dispersion in order to either improve/modify the physical properties of the solid wax particles and/or to allow the solid wax particles to provide other benefits in addition to the benefits obtained from waxes.

### Waxes Having Melting Points of 35°C or Less

Suitable additional waxes that may further comprise the solid wax particle are those waxes whose melting points are at 35°C or less; these waxes include, but are not limited to, Hest 2T-5E-4S, Ditrimethylolpropane tetralaurate, Koster BK-34, Fluoro Polymethylalkyl dimethylsiloxane, Blend of Dilauryl Adipate and Ditetradecyl Adipate, Astrocaryum MuruMuru Seed Butter, Myrica Pubescens Wax, PEG-70 Mango Glycerides, oxypropylenated lanolin wax, hydrogenated Coco-glycerides.

Nevertheless, the waxes whose melting points are at 35°C or less are selected such that the resulting melting point of the solid wax particle of the present disclosure is greater than 35°C.

### Oils

Suitable oils that may comprise the solid wax particle are non-volatile oils, including, but not limited to, mineral oils (paraffin); plant oils and natural oils (sweet almond oil, macadamia oil, grapeseed oil, olive oil, argan oil, tocopherol or vitamin E, shea butter oil, jojoba oil, tocopherol or vitamine E oil); synthetic oils, for instance perhydrosqualene; fatty acids or fatty esters (for instance the C₁₂-C₁₅ alkyl benzoate sold under the trade name Finsolv^{®} TN, commercially available from Innospec or Tegosoft^{®} TN, commercially available from Evonik Goldschmidt, octyl palmitate, isopropyl lanolate; esters such as tocopheryl acetate; and triglycerides, including capric/caprylic acid triglycerides); oxyethylenated or oxypropylenated fatty esters and ethers; or fluoro oils, and polyalkylenes.

Other oils include for example: silicone oils, for instance volatile or non-volatile polymethylsiloxanes (PDMS) with a linear or cyclic silicone chain, which are liquid or pasty at room temperature, especially cyclopolydimethylsiloxanes (cyclomethicones) such as cyclohexasiloxane; polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyltrimethylsiloxydiphenyl-siloxanes, diphenyl dimethicones, diphenylmethyl-diphenyltrisiloxanes or 2-phenylethyl trimethylsiloxy silicates, and polymethylphenylsiloxanes; mixtures thereof.

Other suitable oils include, but are not limited to, hydrocarbon-based oils such as, for example, hydrocarbon oils having from 8 to 16 carbon atoms and their mixtures and in particular branched C₈ to C₁₆ alkanes such as C₈ to C₁₆ isoalkanes (also known as isoparaffins), isododecane, isodecane, isohexadecane, and for example, the oils sold under the trade names of Isopar^{™} or Permethyl^{®}, and their mixtures.

Other suitable oils include esters such as those of formula R₁COOR₂ in which R₁ represents a linear or branched higher fatty acid residue containing from 1 to 40 carbon atoms, including from 7 to 19 carbon atoms, and R₂ represents a branched hydrocarbon-based chain containing from 1 to 40 carbon atoms, including from 3 to 20 carbon atoms, and also including, for example, octyldodecyl neopentanoate, Purcellin oil (cetostearyl octanoate), isononyl isononanoate, C₁₂ to C₁₅ alkyl benzoate, isopropyl myristate, 2-ethylhexyl palmitate, and octanoates, decanoates or ricinoleates of alcohols or of polyalcohols; hydroxylated esters, for instance isostearyl lactate or diisostearyl malate, and pentaerythritol esters. Other suitable esters include polyesters, alkoxylated esters, and alkoxylated polyesters.

The oils may also be chosen from silicones. Suitable silicones include, but are not limited to, the silicone oils described above and other silicones such as non-volatile silicones such as dimethicone fluids having viscosity values of equal to or greater than 300 cst, and pentaphenyldimethicone, also known as trimethyl pentaphenyl trisiloxane, commercially available from Dow Corning under the tradename Dow Corning^{®} 555.

The oil(s) that may further comprise the solid wax particle of the present disclosure is selected such that the melting point of the solid wax particle is greater than 35°C. Preferably, the ratio of oil to wax(es) ranges from between 1:100 to 20:100.

### Emulsifying Polymers

The solid wax particles of the aqueous dispersion of the present disclosure may also comprise an emulsifying polymer, i.e. an amphiphilic polymer.

Among the emulsifying polymers that are suitable for use in the invention, mention may be made of:
POE-POP diblock and triblock copolymers such as those described in patent U.S. Pat. No. 6,464,990;
polyoxyethylenated silicone surfactants such as those described in patent U.S. Pat. No. 6,120,778;
non-crosslinked hydrophobic AMPSs such as those described in EP 1 466 588;
amphiphilic acrylic polymers, such as PEMULEN TR-1 or TR-2 or equivalent;
the associative and gelling polymers described in US 2003/0138465;
heat-gelling polymers such as those described in patent applications US 2004/0214913, US 2003/0147832 and US 2002/0198328 and FR2 856 923.

When they are present, the emulsifying polymer(s) may be introduced in a content ranging from 0.1 percent to 15 percent by weight, or even from 0.1 percent to 10 percent by weight and more particularly from 0.1 percent to 5 percent by weight relative to the total weight of the aqueous dispersion.

### Silicas, Talc, and Clays

The solid wax particle may further comprise sub-micron-sized to micron-sized particles of silica, talc, and/or clays, which include, but are not limited to, montmorillonite, bentonite, hectorite, attapulgite, sepiolite, laponite, smectite, kaolin, and their mixtures.

These clays can be modified with a chemical compound chosen from quaternary ammoniums, tertiary amines, amine acetates, imidazo lines, amine soaps, fatty sulphates, alkylarylsulphonates, amine oxides and their mixtures.

Mention may be made, as organophilic clays, of quaternium-18 bentonites, such as those sold under the names Bentone 3, Bentone 38 or Bentone 38V by Rheox, Tixogel VP by United Catalyst or Claytone 34, Claytone 40 or Claytone XL by Southern Clay; stearalkonium bentonites, such as those sold under the names Bentone 27 by Rheox, Tixogel LG by United Catalyst or Claytone AF or Claytone APA by Southern Clay; or quaternium- 1 8/benzalkonium bentonites, such as those so ld under the names Claytone HT or Claytone PS by Southern Clay.

Suitable silicas may include pyrogenic silicas obtained by high temperature hydrolysis of a volatile silicon compound in an oxyhydrogen flame, producing a finely divided silica. This process makes it possible in particular to obtain hydrophilic silicas which exhibit a large number of silanol groups at their surfaces.

It is possible to chemically modify the surface of the silica by a chemical reaction for the purpose of decreasing the number of silanol groups. It is possible in particular to substitute silanol groups by hydrophobic groups: a hydrophobic silica is then obtained.

The hydrophobic groups can be:
- trimethylsiloxyl groups, which are obtained in particular by treatment of pyrogenic silica in the presence of hexamethyldisilazane. Silicas thus treated are also named "Silica silylate."
- dimethylsilyloxyl or polydimethylsiloxane groups, which are obtained in particular by treatment of pyrogenic silica in the presence of polydimethylsiloxane or of dimethyldichlorosilane. Silicas thus treated are also named "Silica dimethyl silylate."

The pyrogenic silica preferably exhibits a particle size which can be sub-micron sized or micron sized, for example ranging from approximately 5 to 200 nm.

The silica, talc, and/or clays may be present in an amount of from 01% to 10% by weight, or preferably, from 0.5% to 2% by weight, based on the weight of the aqueous dispersion.

### Ceramides

Ceramide compounds that may be useful according to various embodiments of the disclosure include ceramides, glycoceramides, pseudoceramides, and mixtures thereof. The ceramides which may be chosen include, but are not limited to, those described by DOWNING in Arch. Dermatol, Vol. 123, 1381 - 1384 (1987), DOWNING in Journal of Lipid Research, Vol. 35, page 2060 (1994), or those described in French patent FR 2673179.

Further exemplary ceramides that may be used according to various embodiments of the disclosure include, but are not limited to, compounds of the general formula (I): 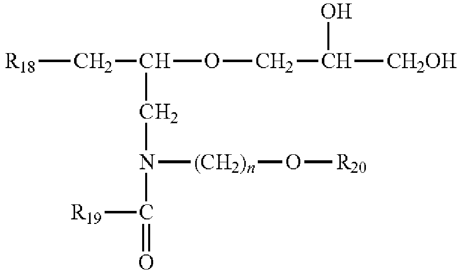 wherein, in formula (I):

-R₁₈ and R₁₉ are, independently, chosen from alkyl- or alkenyl groups with 10 to 22 carbon atoms,
- R₂₀ is chosen from methyl, ethyl, n-propyl or isopropyl groups, and
- n is a number ranging from 1 to 6, such as, for example, 2 or 3.

In further embodiments, ceramide compounds may be chosen from compounds of formula (II), as described in US20050191251 and US20090282623: 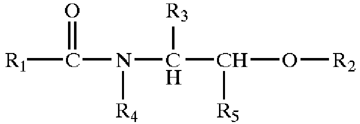 wherein, in formula (II):
-R₁ is chosen from either a saturated or unsaturated, linear or branched C₁-C₅₀, e.g. C₅-C₅₀, hydrocarbon radical, it being possible for this radical to be substituted with one or more hydroxyl groups optionally esterified with an acid R₇COOH, R₇ being an optionally mono- or polyhydroxylated, linear or branched, saturated or unsaturated C₁-C₃₅ hydrocarbon radical, it being possible for the hydroxyl(s) of the radical R₇ to be esterified with an optionally mono- or polyhydroxylated, linear or branched, saturated or unsaturated C₁-C₃₅ fatty acid, or a radical R"--(NR--CO)--R', R being chosen from a hydrogen atom or a mono- or polyhydroxylated, e.g. monohydroxylated, C₁-C₂₀ hydrocarbon radical, R' and R" chosen from, independently, hydrocarbon radicals of which the sum of the carbon atoms is between 9 and 30, R' being a divalent radical, or a radical R₈-O--CO-- (CH₂)p, R₈ denoting a C₁-C₂₀ hydrocarbon radical, p being an integer varying from 1 to 12;
-R₂ being chosen from a hydrogen atom, a saccharide-type radical, in particular a (glycosyl)n, (galactosyl)m and sulphogalactosyl radical, a sulphate or phosphate residue, a phosphorylethylamine radical and a phosphorylethylammonium radical, in which n is an integer varying from 1 to 4 and m is an integer varying from 1 to 8;
-R₃ chosen from a hydrogen atom or a hydroxylated or nonhydroxylated, saturated or unsaturated, C₁-C₃₃ hydrocarbon radical, it being possible for the hydroxyl(s) to be esterified with an inorganic acid or an acid R₇COOH, R₇ having the same meanings as above, and it being possible for the hydroxyl(s) to be etherified with a (glycosyl)n, (galactosyl)m, sulphogalactosyl, phosphorylethylamine or phosphorylethylammonium radical, it being also possible for R₃ to be substituted with one or more C₁-C₁₄ alkyl radicals;
-R₄ being chosen from a hydrogen atom, a methyl or ethyl radical, an optionally hydroxylated, linear or branched, saturated or unsaturated C₃-C₅₀ hydrocarbon radical or a radical --CH₂--CHOH-CH₂--O--R₆ in which R₆ denotes a C₁₀-C₂₆ hydrocarbon radical or a radical R₈--O--CO-- (CH₂) p, R₈ chosen from a C₁-C₂₀ hydrocarbon radical, p being an integer varying from 1 to 12; and
-R₅ denotes a hydrogen atom or an optionally mono- or polyhydroxylated, linear or branched, saturated or unsaturated C₁-C₃₀ hydrocarbon radical, it being possible for the hydroxyl(s) to be etherified with a (glycosyl)n, (galactosyl)m, sulphogalactosyl, phosphorylethylamine or phosphorylethylammonium radical,
-with the proviso that when R₃ and R₅ denote hydrogen or when R₃ denotes hydrogen and R₅ denotes methyl, then R₄ does not denote a hydrogen atom, or a methyl or ethyl radical.

By way of example, ceramides of formula (IV) may be chosen from those wherein R₁ is an optionally hydroxylated, saturated or unsaturated alkyl radical derived from C₁₄-C₂₂ fatty acids; R₂ is a hydrogen atom; and R₃ is an optionally hydroxylated, saturated, linear C₁₁-C₁₇, e.g. C₁₃-C₁₅ radical.

In yet further embodiments, ceramide compounds useful according to the disclosure may be chosen from compounds of the general formula (III): 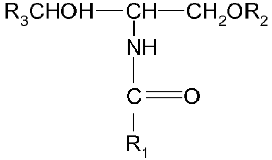 wherein, in formula (III):
- R₁ is chosen from a linear or branched, saturated or unsaturated alkyl group, derived from C₁₄-C₃₀ fatty acids, it being possible for this group to be substituted with a hydroxyl group in the alpha-position, or a hydroxyl group in the omega-position esterified with a saturated or unsaturated C₁₆-C₃₀ fatty acid;
- R₂ is chosen from a hydrogen atom or a (glycosyl)ₙ, (galactosyl)ₘ or sulphogalactosyl group, in which n is an integer ranging from 1 to 4 and m is an integer ranging from 1 to 8; and
- R₃ is chosen from a C₅-C₂₆ hydrocarbon-based group, saturated or unsaturated in the alpha-position, it being possible for this group to be substituted with one or more C₁ - C₁₄ alkyl groups; it being understood that, in the case of natural ceramides or glycoceramides, R₃ may also be chosen from a C₅-C₂₆ alpha-hydroxyalkyl group, the hydroxyl group being optionally esterified with a C₁₆-C₃₀ alpha-hydroxy acid.

Exemplary ceramides of formula (III) which may be chosen include compounds wherin R₁ is chosen from a saturated or unsaturated alkyl derived from C₆-C₂₂ fatty acids; R₂ is chosen from a hydrogen atom; and R₃ is chosen from a linear, saturated C₁₅ group. By way of non-limiting example, such compounds may be chosen from N-linoleoyldihydrosphingosine, N-oleoyldihydrosphingosine, N-palmitoyldihydro-sphingosine, N-stearoyldihydrosphingosine, N-behenoyldihydrosphingosine, or mixtures thereof.

As further non-limiting examples of ceramides, compounds wherein R₁ is chosen from a saturated or unsaturated alkyl group derived from fatty acids; R₂ is chosen from a galactosyl or sulphogalactosyl group; and R₃ is chosen from the group -CH=CH-(CH₂)₁₂-CH₃ group, may be used. In at least one exemplary embodiment, the product consisting of a mixture of these compounds, sold under the trade name Glycocer, by the company Waitaki International Biosciences, may be used.

As further exemplary ceramides, mention may be made of the following ceramides, as described in US20110182839.

In further embodiments, ceramide compounds useful according to the disclosure may be chosen from compounds of the general formula (IV): 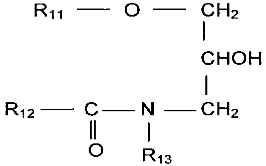 wherein, in formula (IV):
- R₁₁ and R₁₂ are, independently, chosen from alkyl or alkenyl groups with 10 to 22 carbon atoms,
- R₁₃ is an alkyl or hydroxyl alkyl group with 1 to 4 carbon atoms, and
- n is a number ranging from 1 to 6, such as, for example, 2 or 3.

In at least one embodiment, the at least one ceramide compound is chosen from cetyl-PG-hydroxyethylpalmitamide. In a further embodiment, the at least one ceramide compound is chosen from propanediamide, N,N-dihexadecyl-N,N-bis-(2-hydroxyethyl), such as that sold commercially as Questamide H or Pseudoceramide H by the company Quest International Australia Pty. Ltd. In yet a further embodiment, the at least one ceramide compound is chosen from Cetyl-PG Hydroxylpalmatide/decyl glucoside/water, sold as SOFCARE P100H by Kao.

The at least one ceramide compound is present in an amount ranging from 0.001 percent to 20 percent by weight, for example, from 0.01 percent to 10 percent by weight and further for example, from 0.1 percent to 0.5 percent by weight, relative to the total weight of the composition. In one embodiment, the at least one ceramide compound may be present in an amount of 0.5 percent by weight, relative to the total weight of the aqueous dispersion.

### Perfumes

The solid wax particle may further comprise perfumes or fragrances to aid in the fragrance of the product and provide a time-release effect. The perfume can have a dual effect by not only providing a pleasant fragrance but also to provide shine to a treated substrate. The perfumes may be present in an amount of from 0.01% to 10% by weight, or preferably, from 0.5% to 2% by weight, based on the weight of the aqueous dispersion.

### SURFACTANT MIXTURE

The surfactant mixture (b) of the present disclosure comprises (i) at least one non ionic surfactant as defined below and (ii) at least one ionic surfactant comprising at least one specific anionic surfactant.

The nonionic surfactants used in the invention have a Hydrophilic-Lipophilic Balance (HLB) of from at least 5, such as from 5 to 20, or such as from 5 to 15

The at least one nonionic surfactant is chosen from polyethylene glycol ethers of glyceryl esters, sorbitan esters, silicone-based emulsifying polymers having alkoxylated groups and/or side chains, and mixtures thereof. Such nonionic surfactants are disclosed in McCutcheon's "Detergents and Emulsifiers," North American Edition (1986), published by Allured Publishing Corporation; and McCutcheon's "Functional Materials," North American Edition (1992.

Examples of polyethylene glycol ethers of glyceryl esters are PEG-30 glyceryl stearate, PEG-30 glyceryl diisostearate, PEG-30 glyceryl isostearate, PEG-30 glyceryl laurate, and PEG-30 glyceryl oleate .

Preferable sorbitan esters are sorbitan esters of C₁₆-C₂₂ saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN^{®} 80), sorbitan sesquioleate (e.g., Arlacel^{®} 83 from Uniqema, Wilmington, Del.), sorbitan monoisostearate (e.g., CRILL^{®} 6 from Croda, Inc., Edison, N.J.), sorbitan stearates (e.g., SPAN^{®} 60), sorbitan trioleate (e.g., SPAN^{®} 85), sorbitan tristearate (e.g., SPAN^{®} 65), sorbitan palmitate (e.g., SPAN^{®} 40), and sorbitan isostearate. Sorbitan palmitate and sorbitan sesquioleate are particularly preferred for use in the present disclosure.

Particularly preferred nonionic surfactants of the present disclosure are chosen from polyethylene glycol ethers of glyceryl esters such as PEG-30 glyceryl stearate, and sorbitan esters such as sorbitan palmitate.

Other particularly preferred nonionic surfactants are silicone- or siloxane-based emulsifying polymers having alkoxylated groups and/or side chains such as Cetyl PEG/PPG-10/1 Dimethicone (tradename Abil^{®} EM 90); Bis-PEG/PPG-16/16 PEG/PPG-16/16 Dimethicone, commercially available in a mixture with Caprylic/Capric Triglyceride (tradename Abil^{®} Care 85); Bis-PEG/PPG-20/5 PEG/PPG-20/5 Dimethicone and PEG/PPG-25/4 Dimethicone, commercially available in a mixture with Caprylic/Capric Triglyceride (tradename Abil^{®} Care XL 80); Cetyl PEG/PPG-10/1 Dimethicone, commercially available in a mixture with Polyglyceryl-4 Isostearate and Hexyl Laurate (tradename Abil^{®} WE 09); Bis-(Glyceryl/Lauryl) Glyceryl Lauryl Dimethicone, commercially available in a mixture with Caprylic/Capric Triglyceride (tradename Abil^{®} EM 120); Bis-PEG/PPG-14/14 Dimethicone, commercially available in a mixture with dimethicone (tradename Abil EM 97 S), all commercially available from the company, Evonik Goldschmidt GmbH.

Most preferably, (b) (i) is chosen from PEG-30 glyceryl stearate, sorbitan palmitate, Cetyl PEG/PPG-10/1 Dimethicone, Bis-PEG/PPG-16/16 PEG/PPG-16/16 Dimethicone, Bis-PEG/PPG-20/5 PEG/PPG-20/5 Dimethicone, PEG/PPG-25/4 Dimethicone, Bis-(Glyceryl/Lauryl) Glyceryl Lauryl Dimethicone, Bis-PEG/PPG-14/14 Dimethicone, and mixtures thereof.

The nonionic surfactant will typically be employed in an amount of from 60% to 95% by weight, or from 65% to 90% by weight, or from 70% to 90% by weight, based on the total weight of the surfactant mixture of the present disclosure.

Typically, the ionic surfactants contain a lipophilic hydrocarbon group and a polar functional hydrophilic group. The at least one ionic surfactant comprises at least one anionic surfactant chosen from alkyl sulfates and their salts, alkyl ether sulfates and their salts, acyl glutamates, alkyl ether carboxylates, and mixtures thereof.

The following anionic surfactants, which may be used alone or as mixtures, may be mentioned: mention may be made especially of the salts, in particular the alkali metal salts such as the sodium salts, the ammonium salts, the amine salts, the amino alcohol salts or the salts of alkaline-earth metals, for example of magnesium, of the following compounds: alkyl sulfates, alkyl ether sulfates, and acylglutamates, the alkyl or acyl groups of all these compounds comprising from 6 to 24 carbon atoms Among the anionic surfactants that may also be used, mention may also be made of polyoxyalkylenated (C6-C24)alkylether-carboxylic acids, and salts thereof, in particular those comprising from 2 to 50 ethylene oxide groups, and mixtures thereof.

Among the preferred anionic surfactants, mention may be made of the salts, in particular of sodium, of magnesium or of ammonium, of alkyl sulfates; of alkyl ether sulfates, for instance sodium lauryl ether sulfate, preferably containing 2 or 3 mol of ethylene oxide; of acyl glutamates, for instance, disodium stearoyl glutamate and sodium stearoyl glutamate; of alkyl ether carboxylates; and mixtures thereof, the alkyl or acyl groups generally containing from 6 to 24 carbon atoms and preferably from 8 to 16 carbon atoms.

More preferably, the anionic surfactants are chosen from disodium stearoyl glutamate and sodium stearoyl glutamate and mixtures thereof.

The at least ionic surfactant (b) (ii) may further contain at least one cationic surfactant. Among the cationic surfactants, mention may be made of:
i) alkylpyridinium salts, ammonium salts of imidazoline, diquaternary ammonium salts, and ammonium salts containing at least one ester function;
ii) quaternary ammonium salts having the following general formula: 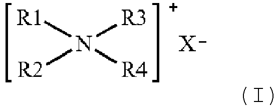 in which the radicals R1 to R4, which may be identical or different, represent a linear or branched aliphatic radical containing from 1 to 30 carbon atoms, or an aromatic radical such as aryl or alkylaryl; the aliphatic radicals may optionally comprise heteroatoms (O, N, S or halogens) and may optionally, be substituted.

The aliphatic radicals are chosen, for example, from C12-C22 alkyl, alkoxy, C2-C6 polyoxyalkylene, alkylamide, (C12-C22)alkylamido(C2-C6)alkyl, (C12-C22)alkylacetate and hydroxyalkyl radicals, containing from 1 to 30 carbon atoms. X- is an anion chosen from the group of halides, phosphates, acetates, lactates, C2-C6 alkyl sulfates and alkyl or alkylarylsulfonates.

iii) quaternary ammonium salts of imidazoline of formula: 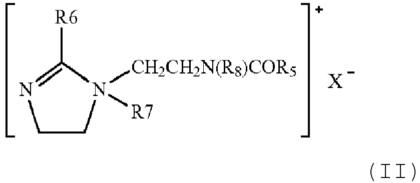 in which:
R5 represents an alkenyl or alkyl radical containing from 8 to 30 carbon atoms, for example fatty acid derivatives of tallow or of coconut,
R6 represents a hydrogen atom, a C1-C4 alkyl radical or an alkenyl or alkyl radical containing from 8 to 30 carbon atoms,
R7 represents a C1-C4 alkyl radical,
R8 represents a hydrogen atom or a C1-C4 alkyl radical,
X' is an anion chosen from the group of halides, phosphates, acetates, lactates, C2-C6 alkyl sulfates, alkylsulfonates or alkylarylsulfonates.
R5 and R6 preferably denote a mixture of alkenyl or alkyl radicals containing from 12 to 21 carbon atoms, such as, for example, fatty acid derivatives of tallow, R7 denotes methyl and R8 denotes hydrogen. Such a product is, for example, Quaternium-27 (CTFA 1997) or Quaternium-83 (CTFA 1997), which are sold under the names Rewoquat(R) W75, W90, W75PG and W75HPG by the company Witco,
iv) diquaternary ammonium salts of formula: 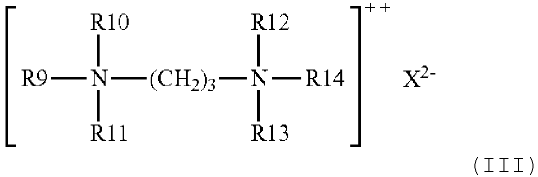
in which:
R9 denotes an aliphatic radical containing from 16 to 30 carbon atoms,
R10, R11, R12, R13 and R14, which may be identical or different, are chosen from hydrogen and an alkyl radical containing from 1 to 4 carbon atoms, and
X- is an anion chosen from the group of halides, acetates, phosphates, nitrates, ethyl sulfates and methyl sulfates.
Such diquaternary ammonium salts in particular comprise propanetallowdiammonium dichloride;
v) quaternary ammonium salts containing at least one ester function, such as those of formula: 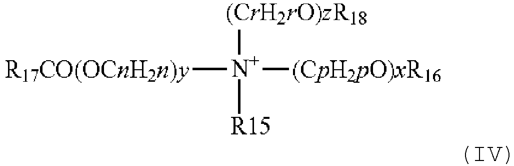
in which:
R15 is chosen from C1-C6 alkyl radicals and C1-C6 hydroxyalkyl or dihydroxyalkyl radicals;
R16 is chosen from the radical R19-CO-, linear or branched, saturated or unsaturated C1-C22 hydrocarbon-based radicals R20, a hydrogen atom;
R18 is chosen from the radical R21-CO, linear or branched, saturated or unsaturated C1-C22 hydrocarbon-based radicals R22, a hydrogen atom;
R17, R19 and R21, which may be identical or different, are chosen from linear or branched, saturated or unsaturated C7-C21 hydrocarbon-based radicals;
r, n and p, which may be identical or different, are integers ranging from 2 to 6;
y is an integer ranging from 1 to 10;
x and z, which may be identical or different, are integers ranging from 0 to 10;
X- is a simple or complex organic or mineral anion;
with the proviso that the sum x+y +z is from 1 to 15, that when x is 0, then R16 denotes R20 and that when z is 0, then R18 denotes R22.

The alkyl radicals R15 may be linear or branched, and more particularly linear. Preferably, R15 denotes a methyl, ethyl, hydroxyethyl or dihydroxypropyl radical, and more particularly a methyl or ethyl radical.

Advantageously, the sum x+y+z is from 1 to 10.

When R16 is a hydrocarbon-based radical R20, it may contain from 12 to 22 carbon atoms, or contain from 1 to 3 carbon atoms.

When R18 is a hydrocarbon-based radical R22, it preferably contains 1 to 3 carbon atoms.

Advantageously, R17, R19 and R21, which may be identical or different, are chosen from linear or branched, saturated or unsaturated C11-C21 hydrocarbon-based radicals, and more particularly from linear or branched, saturated or unsaturated C11-C21 alkyl and alkenyl radicals.

Preferably, x and z, which may be identical or different, are equal to 0 or 1. Advantageously, y is equal to 1.

Preferably, r, n and p, which may be identical or different, are equal to 2 or 3 and even more particularly equal to 2.

The anion X- is preferably a halide (chloride, bromide or iodide) or a C1-C4 alkyl sulfate, more particularly methyl sulfate. The anion X- may also represent methanesulfonate, phosphate, nitrate, tosylate, an anion derived from an organic acid (such as acetate or lactate), or any other anion that is compatible with the ammonium containing an ester function.

The surfactants may be, for example, the salts (chloride or methyl sulfate) of diacyloxyethyldimethylammonium, of diacyloxyethylhydroxyethyldimethylammonium, of monoacyloxyethylhydroxyethyldimethylammonium, of triacyloxyethylmethylammonium, of monoacyloxyethylhydroxyethyldimethylammonium, and mixtures thereof. The acyl radicals preferably contain 14 to 18 carbon atoms and are more particularly derived from a plant oil, for instance palm oil or sunflower oil. When the compound contains several acyl radicals, these radicals may be identical or different. Such compounds are sold, for example, under the names Dehyquart(R) by the company Cognis, Stepanquat(R) by the company Stepan, Noxamium(R) by the company Ceca, and Rewoquat(R)WE 18 by the company Rewo-Goldschmidt.

vi) quaternary ammonium salts and in particular behenyltrimethylammonium chloride, dipalmitoylethyl-hydroxyethylmethylammonium methosulfate, cetyltrimethylammonium chloride, quaternium-83, behenylamidopropyl-2,3-dihydroxypropyl-dimethylammonium chloride and palmitylamidopropyltrimethylammonium chloride.

Other suitable cationic surfactants are esterquats which are quaternary ammonium compounds having fatty acid chains containing ester linkages.

Among the preferred cationic surfactants, mention may be made of compounds of formula (I) chosen from cetrimonium chloride, behentrimonium chloride, Behenyl PG-Trimonium chloride, dicetyl dimonium chloride, and mixtures, thereof.

Other preferred cationic surfactants are esterquats chosen from Dibehenoylethyl Dimonium Chloride, Dipalmitoylethyl Dimonium Chloride, Distearoylethyl Dimonium Chloride, Ditallowoyl PG-dimonium Chloride, Dipalmitoylethyl hydroxyethylmonium methosulfate, Distearoylethyl hydroxyethylmonium methosulfate, and mixtures, thereof.

More preferably, the cationic surfactants are chosen from cetrimonium chloride, behentrimonium chloride, dipalmitoylethyl hydroxyethylmonium methosulfate, distearoylethyl hydroxyethylmonium methosulfate, and mixtures thereof.

Without being bound by any one theory, it is believed that the presence of an ionic surfactant, particularly, at the time of making the dispersion, reduces or minimizes the aggregation of the solid wax particles in the aqueous dispersion of the present disclosure. Thus, the surfactant mixture comprising at least one ionic surfactant acts as a dispersant to facilitate the uniform dispersion of the solid wax particles and to enhance the stabilization of the dispersion itself.

In preferred embodiments, the surfactant mixture contains at least one nonionic surfactant and at least one ionic surfactant comprising at least one anionic surfactant wherein the surfactant mixture is free of cationic surfactants.

The at least one ionic surfactant will typically be employed in an amount of from 5% to 40% by weight, or from 5% to 30% by weight, or from 5% to 20% by weight, based on the total weight of the surfactant mixture of the present disclosure.

Preferably, the surfactant mixture, that is, the combined amount of the at least one nonionic surfactant and the at least one ionic surfactant is present in the aqueous dispersion in an amount of from 1.0% to 5 % by weight, or such as from 1.5% to 3.5 % by weight, or such as from 1.5% to 3% by weight, based on the total weight of the aqueous dispersion.

Those skilled in the art will select the best fit between the wax and surfactant in terms of type and % to get the best dispersions. For example, silicone waxes are generally found to be more compatible with silicone based surfactants.

In certain preferred embodiments, the surfactant mixture of the present disclosure is free of amphoteric surfactants.

Amphoteric surfactants include, but are not limited to, aliphatic secondary or tertiary amine derivatives, in which the aliphatic group is a linear or branched chain containing 8 to 22 carbon atoms and containing at least one water-soluble anionic group, such as, for example, a carboxylate, sulfonate, sulfate, phosphate or phosphonate group; mention may also be made of (C8-C20)alkylbetaines, sulfobetaines, (C8-C20)alkyl-amido-(C6-C8)-alkyl-betaines or (C8-C20)alkyl-amido-(C6-C8)-alkylsulfobetaines; and mixtures thereof.

Among the amine derivatives that may be mentioned are amphocarboxyglycinate compounds and amphocarboxypropionate compounds, in particular, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid, (C8-C20)alkylbetaines, (C8-C20)alkylamido(C6-C8)alkylbetaines and alkylamphodiacetates.

### SUNSCREEN AGENT

The aqueous dipsersions of the present disclosure may also comprise at least one sunscreen agent. Representative sunscreen agents which comprise the aqueous dispersions and compositions of the present invention may be chosen from the organic and inorganic sunscreens or UV filters.

Non-limiting examples of the at least one sunscreen agent include anthranilates; salicylic derivatives; camphor derivatives; benzophenone derivatives; b,b diphenylacrylate derivatives; triazine derivatives; benzotriazole derivatives; benzalmalonate derivatives; benzimidazole derivatives; imidazolines; bis-benzazolyl derivatives as described in patents EP 669 323 and US 2 463 264; p aminobenzoic acid (PABA) derivatives; methylenebis(hydroxyphenylbenzotriazole) derivatives as described in patent applications US 5 237 071, US 5 166 355, GB 2 303 549, DE 197 26 184 and EP 893 119; benzoxazole derivatives such as those described in patent applications EP 0 832 642; EP 1 027 883, EP 1 300 137 and DE 101 62 844; screening polymers and screening silicones such as those described especially in patent application WO 93/04665; dimers derived from a alkyl¬styrene, such as those described in patent application DE 198 55 649; 4,4-diarylbutadienes such as those described in patent applications EP 0 967 200, DE 197 46 654, DE 197 55 649, EP-A-1 008 586, EP 1 133 980 and EP 133 981, and mixtures thereof.

As examples of organic photoprotective agents, mention may be made of those denoted hereinbelow under their INCI name:
para-Aminobenzoic acid derivatives:
PABA,
Ethyl PABA,
Ethyl dihydroxypropyl PABA,
Ethylhexyl dimethyl PABA sold in particular under the name "Escalol 507" by ISP,
Glyceryl PABA,
PEG-25 PABA sold under the name "Uvinul P25" by BASF.

Salicylic derivatives:
Homosalate sold under the name "Eusolex HMS" by Rona/EM Industries,
Ethylhexyl salicylate sold under the name "Neo Heliopan OS" by Haarmann and Reimer,
Dipropylene glycol salicylate sold under the name "Dipsal" by Scher,
TEA salicylate sold under the name "Neo Heliopan TS" by Haarmann and Reimer.

b,b-Diphenylacrylate derivatives:
Octocrylene sold in particular under the trade name "Uvinul N539" by BASF,
Etocrylene sold in particular under the trade name "Uvinul N35" by BASF.

Benzophenone derivatives:
Benzophenone-1 sold under the trade name "Uvinul 400" by BASF,
Benzophenone-2 sold under the trade name "Uvinul D50" by BASF,
Benzophenone-3 or Oxybenzone sold under the trade name "Uvinul M40" by BASF,
Benzophenone-4 sold under the trade name "Uvinul MS40" by BASF,
Benzophenone-5,
Benzophenone-6 sold under the trade name "Helisorb 11" by Norquay,
Benzophenone-8 sold under the trade name "Spectra-Sorb UV-24" by American Cyanamid,
Benzophenone-9 sold under the trade name "Uvinul DS-49" by BASF,
Benzophenone-12
Diethylaminohydroxybenzoylhexyl benzoate sold under the trade name "Uvinul A Plus" by BASF,

Benzylidenecamphor derivatives:
3-Benzylidenecamphor manufactured under the name "Mexoryl SD" by Chimex,
4-Methylbenzylidenecamphor sold under the name "Eusolex 6300" by Merck,
Benzylidenecamphorsulfonic acid manufactured under the name "Mexoryl SL" by Chimex,
Camphor benzalkonium methosulfate manufactured under the name "Mexoryl SO" by Chimex,
Terephthalylidenedicamphorsulfonic acid manufact¬ured under the name "Mexoryl SX" by Chimex,
Polyacrylamidomethylbenzylidenecamphor manufactured under the name "Mexoryl SW" by Chimex.

Phenylbenzimidazole derivatives:
Phenylbenzimidazolesulfonic acid sold in particular under the trade name "Eusolex 232" by Merck,
Disodium phenyl dibenzimidazole tetrasulfonate sold under the trade name "Neo Heliopan AP" by Haarmann and Reimer.

Phenylbenzotriazole derivatives:
Drometrizole trisiloxane sold under the name "Silatrizole" by Rhodia Chimie,
Methylenebis(benzotriazolyl)tetramethylbutyl phenol sold in solid form under the trade name "MIXXIM BB/100" by Fairmount Chemical, or in micronized form as an aqueous dispersion under the trade name "Tinosorb M" by Ciba Specialty Chemicals.

Triazine derivatives:
-Bis(ethylhexyloxyphenol)methoxyphenyl triazine sold under the trade name "Tinosorb S" by Ciba-Geigy,
Ethylhexyl triazone sold in particular under the trade name "Uvinul T150" by BASF,
Diethylhexylbutamidotriazone sold under the trade name "Uvasorb HEB" by Sigma 3V,
2,4,6-tris(diisobutyl 4'-aminobenzalmalonate)-s-triazine.

Anthranilic derivatives:
Menthyl anthranilate sold under the trade name "Neo Heliopan MA" by Haarmann and Reimer.

Imidazoline derivatives:
Ethylhexyldimethoxybenzylidenedioxoimidazoli ne propionate.

Benzalmalonate derivatives:
Polyorganosiloxane containing benzalmalonate functions, for instance Polysilicone-15, sold under the trade name "Parsol SLX" by Hoffmann LaRoche

4,4-Diarylbutadiene derivatives:
1,1-Dicarboxy(2,2'-dimethylpropyl)-4,4-diphenyl¬butadiene

Benzoxazole derivatives:
2,4-bis[5-(1-dimethylpropyl)benzoxazol-2-yl(4-phenyl)iminol-6-(2-ethylhexyl)imino-1,3,5-triazine sold under the name Uvasorb K2A by Sigma 3V
and mixtures thereof.

Examples of mineral photoprotective agents are chosen from pigments and even more preferably nanopigments (mean size of the primary particles: generally between 5 nm and 100 nm and preferably between 10 nm and 50 nm) of treated or untreated metal oxides such as, for example, nanopigments of titanium oxide (amorphous or crystallized in rutile and/or anatase form), of iron oxide, of zinc oxide, of zirconium oxide or of cerium oxide.

The treated nanopigments are pigments that have undergone one or more surface treatments of chemical, electronic, mechanochemical and/or mechanical nature with compounds as described, for example, in Cosmetics & Toiletries, February 1990, Vol. 105, pp. 53-64, such as amino acids, beeswax, fatty acids, fatty alcohols, anionic surfactants, lecithins, sodium, potassium, zinc, iron or aluminium salts of fatty acids, metal (titanium or aluminium) alkoxides, poly¬ethylene, silicones, proteins (collagen or elastin), alkanolamines, silicon oxides, metal oxides, sodium hexametaphosphate, alumina or glycerol.

The treated nanopigments may more particularly be titanium oxides treated with:
- silica and alumina, such as the products "Micro¬titanium Dioxide MT 500 SA" and "Microtitanium Dioxide MT 100 SA" from the company Tayca, and the products "Tioveil Fin", "Tioveil OP", "Tioveil MOTG" and "Tioveil IPM" from the company Tioxide,
- alumina and aluminium stearate, such as the product "Microtitanium Dioxide MT 100 T" from the company Tayca,
- alumina and aluminium laurate, such as the product "Microtitanium Dioxide MT 100 S" from the company Tayca,
- iron oxides and iron stearate, such as the product "Microtitanium Dioxide MT 100 F" from the company Tayca,
- silica, alumina and silicone, such as the products "Microtitanium Dioxide MT 100 SAS", "Microtitanium Dioxide MT 600 SAS" and "Microtitanium Dioxide MT 500 SAS" from the company Tayca,
- sodium hexametaphosphate, such as the product "Microtitanium Dioxide MT 150 W" from the company Tayca,
- octyltrimethoxysilane, such as the product "T-805" from the company Degussa,
- alumina and stearic acid, such as the product "UVT M160" from the company Kemira,
- alumina and glycerol, such as the product "UVT-M212" from the company Kemira,
- alumina and silicone, such as the product "UVT-M262" from the company Kemira.

Other titanium oxide nanopigments treated with a silicone are preferably TiO2 treated with octyltrimethylsilane and for which the mean size of the elementary particles is between 25 and 40 nm, such as the product sold under the trade name "T805" by the company Degussa Silices, TiO2 treated with a polydimethylsiloxane and for which the mean size of the elementary particles is 21 nm, such as the product sold under the trade name "70250 Cardre UF TiO2SI3" by the company Cardre, anatase/rutile TiO2 treated with a polydimethylhydrogenosiloxane and for which the mean size of the elementary particles is 25 nm, such as the product sold under the trade name "Microtitanium Dioxide USP Grade Hydrophobic" by the company Color Techniques.

The uncoated titanium oxide nanopigments are sold, for example, by the company Tayca under the trade names "Microtitanium Dioxide MT 500 B" or "Microtitanium Dioxide MT 600 B", by the company Degussa under the name "P 25", by the company Wackher under the name "Oxyde de titane transparent PW", by the company Myoshi Kasei under the name "UFTR", by the company Tomen under the name "ITS" and by the company Tioxide under the name "Tioveil AQ".

The uncoated zinc oxide nanopigments are, for example:
- those sold under the name "Z-Cote" by the company Sunsmart;
- those sold under the name "Nanox" by the company Elementis;
- those sold under the name "Nanogard WCD 2025" by the company Nanophase Technologies.

The coated zinc oxide nanopigments are, for example:
- those sold under the name "Zinc Oxide CS-5" by the company Toshibi (ZnO coated with polymethylhydrogenosiloxane) ;
- those sold under the name "Nanogard Zinc Oxide FN" by the company Nanophase Technologies (as a 40% dispersion in Finsolv TN, C12-C15 alkyl benzoate);
- those sold under the name "Daitopersion ZN-30" and "Daitopersion ZN-50" by the company Daito (dispersions in cyclopolymethylsiloxane/oxyethylenated polydimethyl¬siloxane, containing 30% or 50% of nanozinc oxides coated with silica and polymethylhydrogenosiloxane);
- those sold under the name "NFD Ultrafine ZNO" by the company Daikin (ZnO coated with perfluoroalkyl phosphate and copolymer based on perfluoroalkylethyl as a dispersion in cyclopentasiloxane);
- those sold under the name "SPD-Z1" by the company Shin-Etsu (ZnO coated with silicone-grafted acrylic polymer, dispersed in cyclodimethylsiloxane);
- those sold under the name "Escalol Z100" by the company ISP (alumina-treated ZnO dispersed in an ethylhexyl methoxycinnamate/PVP-hexadecene/methicone copolymer mixture);
- those sold under the name "Fuji ZNO-SMS-10" by the company Fuji Pigment (ZnO coated with silica and polymethylsilsesquioxane);
- those sold under the name "Nanox Gel TN" by the company Elementis (ZnO dispersed at a concentration of 55% in C12-C15 alkyl benzoate with hydroxystearic acid polycondensate).

The uncoated cerium oxide nanopigments are sold under the name "Colloidal Cerium Oxide" by the company Rhone-Poulenc.

The uncoated iron oxide nanopigments are sold, for example, by the company Arnaud under the names "Nanogard WCD 2002 (FE 45B)", "Nanogard Iron FE 45 BL AQ", "Nanogard FE 45R AQ" and "Nanogard WCD 2006 (FE 45R)" or by the company Mitsubishi under the name "TY-220",

The coated iron oxide nanopigments are sold, for example, by the company Arnaud under the names "Nanogard WCD 2008 (FE 45B FN)", "Nanogard WCD 2009 (FE 45B 556)", "Nanogard FE 45 BL 345" and "Nanogard FE 45 BL" or by the company BASF under the name "Transparent Iron Oxide".

Mention may also be made of mixtures of metal oxides, especially of titanium dioxide and of cerium dioxide, including the silica-coated equal-weight mixture of titanium dioxide and of cerium dioxide, sold by the company Ikeda under the name "Sunveil A", and also the alumina, silica and silicone-coated mixture of titanium dioxide and of zinc dioxide, such as the product "M 261" sold by the company Kemira, or the alumina, silica and glycerol-coated mixture of titanium dioxide and of zinc dioxide, such as the product "M 211" sold by the company Kemira.

The nanopigments may be introduced into the compositions according to the invention in unmodified form or in the form of pigmentary paste, i.e. as a mixture with a dispersant, as described, for example, in document GB A 2 206 339.

One particularly preferred sunscreen agent of the present invention is octocrylene.

Other particularly preferred sunscreen agents of the present invention are chosen from Terephthalylidene dicamphor derivatives, benzylidenecamphor derivatives and benzotriazole derivatives, in particular, drometrizole trisiloxane, also known under the tradename of Mexoryl XL.

When the sunscreen agent is present, its amount ranges typically from 0.1% to 10% by weight, such as from 0.1% to 8% by weight, and from 0.5% to 5% by weight, based on the total weight of the aqueous dispersion.

### VOLATILE SOLVENTS

The aqueous dispersions of the present disclosure may also comprise volatile solvents.

In this embodiment, the aqueous dispersions and compositions containing the aqueous dispersion of the present disclosure form a film or coating on the surface of chair wherein the film or coating exhibited better adhesion as the volatile solvent evaporated over time and is resistant to water and/or rubbing.

Although not wanting to be bound by any particular theory, it is believed that the presence of the volatile solvent in the aqueous wax dispersion of the present disclosure helps to soften the wax and make it more pliable, thereby making it easier to apply on chair.

Representative examples of suitable volatile organic solvents include, but are not limited to, volatile hydrocarbon-based oils and volatile silicone oils.

The expression "hydrocarbon-based oil" means oil containing only hydrogen and carbon atoms.

Suitable volatile hydrocarbon oils include, but are not limited to, those having from 8 to 16 carbon atoms and their mixtures and in particular branched C8 to C16 alkanes such as C8 to C16 isoalkanes (also known as isoparaffins), isododecane, isodecane, isohexadecane, and for example, the oils sold under the trade names of Isopar or Permethyl, the C8 to C16 branched esters such as isohexyl or isodecyl neopentanoate and their mixtures. Preferably, the volatile hydrocarbon oils have a flash point of at least 40 degrees centigrade. It is also possible to use mixtures of isoparaffins. Other volatile hydrocarbon-based oils, such as petroleum distillates, can also be used.

Suitable volatile silicone oils include linear or cyclic silicone oils having a viscosity at room temperature less than or equal to 6 cSt and having from 2 to 7 silicon atoms, these silicones being optionally substituted with alkyl or alkoxy groups of 1 to 10 carbon atoms. Examples of volatile silicone oils that may be used include, but are not limited to, octamethyltetrasiloxane, decamethylcyclopentasiloxane, dodecamethylcyclohexasiloxane, heptamethyloctyltrisiloxane, hexamethyldisiloxane, decamethyltetrasiloxane, dodecamethylpentasiloxane, and their mixtures. Preferably, the volatile silicone oils have a flash point of at least 40 degrees centigrade.

Other suitable volatile solvents may be chosen from polar volatile solvents is desired. Such solvents may include, but are not limited to, alcohols, volatile esters and volatile ethers. In general, they will have a flash point below 25 degrees centigrade.

A particularly preferred volatile solvent of the present disclosure is isododecane (also known as 2,2,4,4,6-pentamethylheptane) .

When the volatile solvent is present, its amount generally ranges from 0.1% to 20%, and in some embodiments from 0.5% to 15%, and in some embodiments from 1% to 10%, by weight, based on the total weight of the composition.

In a particular embodiment of the invention, the aqueous dispersion comprises:
(a) at least one solid wax particle having a particle size ranging from 5 microns to 25 microns and comprising at least one wax chosen from beeswax, hydrogenated myristyl olive esters, hydrogenated stearyl olive esters, VP/eicosene copolymer, ditrimethyloylpropane tetrastearate, and C30-45 alkyldimethylsilyl propylsilsesquioxane, and wherein the at least one wax is present in an amount of from 20% to 40% by weight, based on the total weight of the aqueous dispersion;
(b) from 1.5% to 3.0 % by weight of a surfactant mixture comprising:
   (i) at least one nonionic surfactant chosen from PEG-30 glyceryl stearate, sorbitan palmitate, Cetyl PEG/PPG-10/1 Dimethicone, Bis-PEG/PPG-16/16 PEG/PPG-16/16 Dimethicone, Bis-PEG/PPG-20/5 PEG/PPG-20/5 Dimethicone, PEG/PPG-25/4 Dimethicone, Bis-(Glyceryl/Lauryl) Glyceryl Lauryl Dimethicone, Bis-PEG/PPG-14/14 Dimethicone, and mixtures thereof; and
   (ii) from 5% to 20% by weight, based on the total weight of the surfactant mixture, of at least one anionic surfactant chosen from dipalmitoylethyl hydroxyethylmonium methosulfate, distearoylethyl hydroxyethylmonium methosulfate, disodium stearoyl glutamate and sodium stearoyl glutamate, and mixtures thereof; wherein the surfactant mixture is free of amphoteric surfactants;
(c) water; and
(d) optionally, at least one sunscreen agent present in an amount of from 0.1% to about 6% by weight, based on the total weight of the aqueous dispersion;
(e) optionally, from 1% to 10% by weight, based on the total weight of the aqueous dispersion, of at least one volatile solvent comprising isododecane; and
(f) optionally, at least one additional ingredient chosen from a wax having a melting point of 35°C or less, oils, emulsifying polymers, pigments/ dyes, silicas, talc, clays, ceramides and perfumes.

### Process for Obtaining the Aqueous Dispersions (Wax Dispersion Protocol)

The aqueous dispersions of the present disclosure may be obtained by means of a process comprising at least the steps as follow:
emulsifying a mixture containing at least one wax having a melting point or melting temperature greater than 35°C, a surfactant mixture comprising a nonionic surfactant and an ionic surfactant, as defined above, and water at an emulsification temperature above the melting point of the at least one wax. If two or more waxes are used, the emulsification temperature should be higher than the melting point of the wax with the higher or highest melting point,
subjecting the mixture to a process leading to the production of solid wax particles, at a temperature at least 5 to 10°C above the emulsification temperature of the mixture used in the preceding step, and
cooling the dispersion thus obtained.

It is pointed out that the combination of ingredients in the first step of the process and the execution of the second step with heating are cumulative conditions necessary for obtaining the solid wax particles according to the invention in a controlled manner, resulting in solid wax particles that are calibrated to certain properties (e.g., melting point, size, and shape). Thus, the nature of the process exerted on the wax-surfactant-water mixture determines the properties of the particles to be obtained.

The process according to the invention may, where appropriate, also include a step consisting in diluting the continuous phase of the mixture before the cooling step.

For the purposes of the present invention, the expression "process leading to the production of solid wax particles" is intended to denote an action of shear type. This shearing action can be accomplished by mixing the wax-surfactant-water mixture using a homogenizer/mixer at a specified speed.

For example, by using different speeds of mixing, different particle sizes can be achieved such as those ranging from 0.5-100 microns, 1-50 microns, 2-25 microns, 8-20 microns, 2-10 microns, and even less than 1 micron. It is also possible to use other shearing processes such as those described and referred to in US2006/0292095 and US2006/0263438.

The amounts and the types of surfactants in and/or the weight ratios of the surfactants to one another the surfactant mixture and/or the amounts and/or types of waxes employed may also result in wax particles of different particle sizes such as those listed above.

The emulsification temperature is preferably greater than 40 degrees C. and preferably less than 95 degrees C.

Thus, in accordance with the process above, the dispersions of the present disclosure comprise solid wax particles that are calibrated to specific properties.

In one particular embodiment, these particles are free of volatile solvent.

Furthermore, in accordance with the process above, other ingredients, such as active ingredients, polymers, and other additional ingredients as described above may be added during the preparation of the dispersion.

### Dispersion

In accordance with the process described above, the solid wax part particles are preferably obtained as a dispersion in a aqueous and/or water-soluble continuous phase. Such a dispersion may also be described as an oil-in-water emulsion or an oil-in-water dispersion.

The solid wax particles in accordance with the invention advantageously do not aggregate in the dispersion in which they are obtained, and their granulometric specificities in terms of size and distribution index are advantageously conserved therein.

The aqueous and/or water-soluble continuous phase that is suitable for use in the invention preferably comprises water or a combination of water and a water-soluble organic solvent.

Among the water-soluble solvents that may be used in the dispersions in accordance with the invention, mention may be made especially of lower monoalcohols containing from 1 to 5 carbon atoms, such as ethanol and isopropanol, monoalcohols containing from 8+ carbon atoms, glycols, glycol ethers, and polyols, for instance glycerol, ethylene glycol, propylene glycol, butylene glycol, caprylyl glycol, hexylene glycol, dipropylene glycol, diethylene glycol, xylitol, sorbitol, mannitol, maltitol, and polyethylene glycol or mixtures thereof, C3 and C4 ketones, and C2-C4 aldehydes and mixtures thereof.

For the purposes of the present invention, the term "water-soluble solvent" is intended to denote a compound that is liquid at room temperature and water-miscible (miscibility in water of greater than 50 percent by weight at 25°C and at atmospheric pressure).

According to yet another embodiment variant, the dispersions in accordance with the present invention may comprise demineralized water as the continuous aqueous phase.

In the cosmetic, dermatology, personal care and pharmaceutical field, the
dispersions in accordance with the present invention may be used as vehicles for at least one active substance for the preparation of (a) cosmetic and/or dermatological and/or personal care and/or pharmaceutical composition(s).

Thus, the subject of the present invention is compositions, such as cosmetic or dermatological or personal care or pharmaceutical compositions, comprising at least one dispersion as defined above, and a carrier.

More particularly, the invention relates to a hair styling composition comprising an aqueous dispersion according to the invention and at least one cosmetically acceptable carrier.

The aqueous dispersions of the present disclosure is formulated into compositions of various galenic forms.

The aqueous dispersion is employed in a composition such that the amount of the at least one wax comprising the solid wax particle of the aqueous dispersion is from 1% to 5% by weight, or preferably, from 1.5% to 5% by weight, such as from 2% to 5% by weight, relative to the total weight of the composition.

The compositions containing the aqueous dispersions of the present disclosure comprise a carrier chosen from water, volatile and non-volatile organic solvents, silicones, polyols, glycols, glycol ethers, oils, and mixtures thereof.

When the organic solvent is a volatile solvent, the amount of the volatile organic solvent generally ranges from greater than 0 (e.g., 0.01%) to 99%, and in some embodiments from greater than 0 to 55%, and in some embodiments from greater than 0 to 2%, by weight, based on the total weight of the composition. In certain embodiments, the amount of volatile organic solvent does not exceed 55%.

In preferred embodiments, the carrier is a cosmetically, dermatologically or physiologically acceptable carrier that is non toxic, wherein the compositions can be applied onto hair, . The cosmetically, dermatologically or physiologically acceptable carrier may comprise water and/or one or more of the organic solvents, silicones, polyols, glycols, glycol ethers, oils.

The carrier can be employed in an amount of from 70% to 99% by weight, or such as from 75% to 95% by weight, or such as from 80% to 90% by weight, based on the total weight of the composition.

### AUXILIARY AGENT

The compositions comprising the aqueous dispersion of the present disclosure may additionally contain an auxiliary agent chosen from liquid lipids/oils, film forming polymers, rheology modifiers, sunscreen agents, pigments, dyes, silica, clays, humectants and moisturizing agents, emulsifying agents, structuring agents, propellants, surfactants, shine agents, conditioning agents, cosmetically, dermatologically and pharmaceutically active agents, vitamins, and plant extracts.

### LIQUID LIPIDS/OILS

Representative liquid lipids comprise oils, triglycerides and liquid fatty substances such as mineral oil, avocado oil, camellia oil, turtle oil, macadamia nut oil, corn oil, mink oil, olive oil, rape seed oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, chinese-wood oil, japanese-wood oil, jojoba oil, germ oil, triglycerol, glyceryl trioctanoate, pentaerythritol tetraoctanote, and glyceryl triisopalmitate.

### FILM FORMING POLYMERS

The term "film forming polymer" means a polymer capable, by itself or in the presence of an auxiliary film-forming agent, of forming a continuous film that adheres to a support and especially to keratin materials. Among the film-forming polymers that may be used, mention may be made of synthetic polymers, of free-radical type or of polycondensate type, polymers of natural origin and mixtures thereof, in particular acrylic polymers, polyurethanes, polyesters, polyamides, polyureas and cellulose-based polymers, for instance nitrocellulose.

### RHEOLOGY MODIFIERS

Representative rheology modifiers include, but are not limited to, thickening agents, and gelling agents.

Broadly, the rheology modifier (s) that may be useful in the practice of the present invention include those conventionally used in cosmetics such as polymers of natural origin and synthetic polymers, including, but not limited to, associative polymers, non-associative thickening polymers, and water-soluble thickening polymers.

Representative rheology-modifiers that may be used in the practice of the present invention may be chosen from nonionic, anionic, cationic, and amphoteric polymers, including acrylate- or acrylic-based polymers, polysaccharides, polyamino compounds, and nonionic, anionic, cationic and amphoteric amphiphilic polymers.

Sutiable rheology modifiers include but are not limited to, acrylates copolymers and carbomers. Other suitable rheology modifiers include, but are not limited to, cellulose-based thickeners (*e.g.,* hydroxyethylcellulose, hydroxypropylcellulose, carboxymethylcellulose, cationic cellulose ether derivatives, quaternized cellulose derivatives, etc.), guar gum and its derivatives (e.g., hydroxypropyl guar, cationic guar derivatives, etc.), gums such as gums of microbial origin (e.g., xanthan gum, scleroglucan gum, etc.), and gums derived from plant exudates (e.g., gum arabic, ghatti gum, karaya gum, gum tragacanth, carrageenan gum, agar gum and carob gum), pectins, alginates, and starches, crosslinked homopolymers of acrylic acid or of acrylamidopropane-sulfonic acid.

The rheology modifiers of the present disclosure may also be used as film forming agents in the compositions and aqueous dispersions of the present disclosure, depending on the amount employed.

Examples of rheology modifiers of the present disclosure are polyacrylate-3, commercially known under the trade name of Viscophobe DB-100 and commercially available from The Dow Chemical Company, carbomers, commercially known under the trade name of Carbopol polymers and commercially available from Lubrizol Advance Materials, Inc, acrylates/C10-30 alkyl acrylate crosspolymers, commercially known the trade names of Pemulen TR-1 and Pemulen TR-2 polymers and commercially available from Lubrizol Advance Materials, Inc, AMP-acrylates/allyl methacrylate copolymer, commercially known under the trade name of Fixate G-100 polymer and commercially available from Lubrizol Advance Materials, Inc and polyvinylpyrrolidone, commercially known under the trade name of PVP and commercially available from International Specialty Porducts.

The rheology modifier is typically present in an amount ranging from 0.01% to 10% by weight, in some embodiments from 0.1% to 5% by weight, based on the total weight of the composition.

SUNSCREEN AGENTS, PIGMENTS, DYES, SILICA AND CLAYS

Representative sunscreen agents which comprise the solid wax particle as described above may also comprise the compositions of the present disclosure.

The pigments and dyes, silicas and clays described above which may comprise the solid wax particle of the aqueous dispersion may also comprise the compositions of the present disclosure.

### HUMECTANTS AND MOISTURIZING AGENTS

Suitable examples of humectants and moisturizing agents include, but are not limited to urea, hydroxyethyl urea, polyols such as glycerin, and glycosaminoglycans (GAGs). Suitable examples of glycosaminoglycans are hyaluronic acid or hyaluronan (HA), heparan sulfate (HS), heparin (HP), chondroitin, chondroitin sulfate (CS), chondroitin 4-sulfate or chondroitin sulfate A (CSA), chondroitin 6-sulfate or chondroitin sulfate C (CSC), dermatan sulfate or chondroitin sulfate B (CSB) and keratan sulfate (KS).

### PROPELLANTS

Representative examples of propellants include n-butane, isobutane, propane, dimethyl ether (available commercially from Harp Int'l under the tradename HARP DME), C2-C5 halogenated hydrocarbons, e.g., 1,1-difluoroethane (available commercially from DuPont under the tradename DYMEL 152a), difluoroethane, chlorodifluoroethane, dichlorodifluoromethane, chlorodifluoromethane, trichlorofluoromethane, and mixtures thereof. The amount of the propellant generally ranges from 1 to 55%, and in some embodiments from 1 to 35%, by weight, and in some embodiments from 1 to 20%, by weight and in some embodiments from 2 to 15%, by weight based on the total weight of the composition.

### SURFACTANTS

The surfactants that may be employed as auxiliary agents may be chosen from anionic, cationic, nonionic and amphoteric surfactants such as those described above.

### SHINE AGENTS

The shine agents may be chosen from silicones, alkoxylated silicones, oils, ethoxylated oils, fats, esters, transesters, hydrocarbons, quats and mixtures thereof.

Non-limiting examples of shine agents include Amodimethicone, Dimethicone, Dimethiconol, Cyclemethicone, Phenyltrimethicone, Aminopropyl Phenyltrimethicone, Trimethyl Pentaphenyl Trisiloxane, Cetyl Dimethicone, Alkyl Dimethicone, Potassium Dimethicone PEG-7 Pantheyl Phosphate, Olive oil, Jojoba oil, Apricot oil, Avocado oil, Castor oil, Lanolin, Squalane, Capric/Caprylic Triglyceride, Octyl Palmitate, Isopropyl Palmitate, Isopropyl Myristate, Mineral oil, Petrolatum, Polyquaternium-4, Polyquaternium-11, Behentrimonium Methosulfate, Benetrimonium Chloride and mixtures thereof.

The aqueous dispersions of the present disclosure may additionally comprise one or more additives chosen from pearlescent agents, opacifying agents, fragrances, sequestering agents, softeners, antifoams, wetting agents, spreading agents, dispersants, plasticizers, mineral fillers, colloidal minerals, peptizers, preserving agents, and pH adjusters.

The compositions comprising the aqueous dispersions of the present disclosure may be in the form of an aqueous system, a simple or complex emulsion (oil-in-water (o/w), water-in-oil (w/o), silicone-in-water and / or water-in-silicone emulsion types) such as a cream or a milk, in the form of a gel or a cream-gel, or in the form of a lotion, a powder or a solid tube, and may optionally be packaged as an aerosol and may be in the form of a mousse or a spray. The mousse or spray may contain propellants such as those listed above.

Spray compositions, especially aerosols, typically contain at least one volatile organic compound (VOC). For essentially ecological reasons and governmental regulations in various countries, it is sought or even necessary to reduce the amount of volatile organic compounds (VOCs) present in the composition. To reduce the amount of VOC and to obtain a low-VOC aerosol device, the organic solvents, for instance ethanol and dimethyl ether, are partially replaced with water.

When the compositions of the present disclosure are emulsions, they will generally contain at least one emulsifier/surfactant chosen from amphoteric, anionic, cationic and nonionic emulsifiers or surfactants, which are used alone or as a mixture. The emulsifiers are appropriately chosen according to the emulsion to be obtained.

In another embodiment of the invention, the subject compositions are formulated as water-in-silicone (W/Si) or silicone-in-water (Si/W) emulsions in which the continuous oily phase comprises at least one silicone oil. When the compositions of the invention are formulated as water-in-silicone emulsions, the silicone oils are preferably present in a proportion of at least 5 percent and preferably ranging from 10 percent to 45 percent by weight with respect to the total weight of the emulsion. The fatty phase of the water-in-oil emulsions according to the invention can additionally comprise one or more hydrocarbon-comprising oil(s) in a proportion preferably ranging up to 40 percent by weight with respect to the total weight of the fatty phase of the emulsion.

For the W/Si emulsions, examples of emulsifiers generally include polyether-modified silicones having a long chain of dimethyl siloxane units which carry polyethoxy-polypropoxy units in the chain and at the ends. Examples include cyclopentasiloxane PEG/PPG-18/18 dimethicone, PEG-12 Dimethicone, and PEG/PPG-19/19 Dimethicone sold by Dow Corning under the name Dow Corning^{®} BY 11-030.

The compositions of the present disclosure are applied onto hair

The hair styling compositions of the present disclosure are applied onto hair in order to impart a shape or re-positionability or re-shapeability properties to the hair.

In other embodiments, the application of an external stimuli such as heat onto treated hair, may be desirable or required in order to impart additional benefits to the treated chair.

Thus, in certain embodiments, a method of coating hair is provided, wherein said method involves applying onto the hair, the aqueous dispersion of the present disclosure and a carrier, and heating the hair in order to melt the solid wax particles in the aqueous dispersion. In order to melt the solid wax particle, the heat applied to the hair has to be at a temperature greater than the melting point of the solid wax particles or of the wax that has the highest melting point, if two or more waxes comprise the solid wax particle.

Another embodiment relates to a hair shaping method comprising the method of coating hair. The hair may then be shaped or positioned to achieve a certain style or appearance.

The above-described method allows one to shape/re-shape or re-position the hair on the head, such as to straighten the hair, curl the hair, redefine hair curl, or volumize the hair, and to repeat the steps of said method as many times as desired and without needing to re-apply the composition and/or re-wet the hair.

In particularly preferred embodiments, a means for shaping the hair is used. The means for physically shaping the hair may be part of the heating tool or may be a separate device or tool such as a brush or comb or curling device. The means for physically shaping the hair may also comprise passing the fingers or the hand through the hair.

The steps of the above-described method for shaping hair may be conducted in any order. For example, the composition containing the aqueous dispersion may first be applied onto hair, followed by heating the hair, then followed by shaping the hair using a means for shaping the hair. In another example, the hair may be heated first, followed by the step of applying the composition onto the hair, then followed by the step of shaping the hair using a means for shaping the hair. In yet another example, the hair may be shaped first, using a means for shaping the hair, followed by applying the composition onto the hair and then heating the hair. In other examples, the hair may be shaped first using a means for shaping the hair, followed by heating the hair and then applying the composition onto the hair and allowing the shape of the hair to set in place as the temperature reaches room temperature.

The compositions of the present invention may especially constitute hair styling/shaping, hair straightening, hair waving/curling, hair care, hair treatment, hair conditioning and hair shampoo products.

Professional and consumer heating tools can be used as a means to deliver heat or an elevated temperature to the hair. The heating tools can generate heat through electrical current or heating lamps. Depending upon the desired style, these tools include, but are not limited to, heaters, blow dryers, flat irons, hot combs, hot curler sets, steam pods, heated crimpers, heated lash curlers, heated wands/brushes, and hood driers or their combinations thereof.

In yet other embodiments, compositions containing the aqueous dispersion of the present disclosure are heat-activated in order to allow the compositions to provide additional benefits to hair which has been treated with the composition.

The term "heat-activated" means that heat is used as a stimulus to melt the solid wax particles in the aqueous dispersion.

The hair may be heated or exposed to heat before or after treating the hair with the composition containing the aqueous dispersion of the present disclosure. The hair may also be molded or shaped or positioned as desired while being heated or exposed to heat.

The compositions containing the aqueous dispersion of the present disclosure may especially constitute cosmetic, personal care, dermatological, pharmaceutical products such as hair color, hair styling, hair care and hair cleansing products, suncare, and makeup products.

The compositions of the present invention can be provided in a plethora of galenic forms, including but not limited to creams, liquid, gel, cream-gel, lotion, foam, serum, paste, semi-solid, solid stick, stick-gel, or a powder, and may be in the form of a mousse or a spray, and may optionally be packaged as an aerosol, prepared according to the usual methods.

The following examples of dispersions and of compositions are intended to illustrate the invention without limiting the scope as a result. The percentages are given on a weight basis.

### EXAMPLES

### Example I

Based on the Wax Dispersion Protocol described above, aqueous wax dispersions were individually prepared/manufactured as follow:
A. Aqueous surfactant solution:
   1. A surfactant mixture was prepared by adding gram amounts of nonionic surfactant(s) and ionic surfactant(s) in a container.
   2. A preservative was added to the surfactant mixture.
   3. Deionized water was added in an amount such that the final weight of the aqueous dispersion (including the weight of the wax) is 100 grams.
   4. The surfactant solution was heated to 75°C in a water bath.
B. Oil: A weighed amount of the wax (e.g., beeswax or phyto olive wax) was heated and melted for a few minutes, e.g., 5 minutes, in a microwave (or other appropriate heating device).
C. Emulsification process
   1. While the aqueous surfactant solution was still at an elevated temperature (above room temperature, such as from 65°C to 70°C), the solution was mixed using a homogenizer/mixer (e.g., Silverson homogenizer) at a speed ranging from 3000 to 9000 rpm until bubbles were observed.
   2. The melted hot wax was added to the surfactant solution close to the mixing head of the homogenizer while mixing.
   3. Once all the wax was added, mixing was continued for at least 5 minutes.
   4. The homogenizer blade was removed and the wax emulsion (dispersion) was mixed and cooled slightly towards room temperature before transferring into another container.
   5. The dispersion was stored at room temperature.
   6. The procedure above was followed for preparing other aqueous wax dispersions of the present invention using different waxes and/or surfactants at different levels.
   7. The particle sizes of the solid wax particles were measured using image analysis (microscopy) and/or laser diffraction methods and/or by particle size analyzer to obtain an average particle size. For example, solid wax particles of beeswax were found to have a particle size distribution ranging from 10 to 17 microns while solid wax particles of VP/eicosene copolymer were found to have a particle size distribution ranging from 1 to 11 microns as measured by a Shimadzu SALD-7001 laser diffraction particle size analyzer, using quartz tubes having a refractive index of 1.2.

**Table 1: Examples of aqueous dispersions containing beeswax individually prepared according to the Wax Dispersion Protocol above**

| Ingredient | Aqueous Dispersions % weight of ingredient | | | | |
|---|---|---|---|---|---|
| | A | B | C | D | |
| Beeswax | 30 | 30 | 30 | 30 | |
| PEG-30 glyceryl stearate (nonionic surfactant) | 2.5 | 1.25 | 2.7 | 1.25 | |
| Monosodium stearoyl glutamate (anionic surfactant) | 0.5 | 0.25 | - | - | - |
| Disodium stearoyl glutamate (anionic surfactant) | - | - | 0.3 | 0.25 | - |
| Cetrimonium Chloride (cationic surfactant) | - | - | - | - | |
| phenoxyethanol (preservative) | 0.5 | 0.5 | 0.5 | 0.5 | |
| Deionized Water | Q.S. to 100 | Q.S. to 100 | Q.S. to 100 | Q.S. to 100 | |
| Speed of mixing | 3000 | 4000 | 6000 | 6000 | |
| Solid wax particle size range by image analysis* (microns or µ) | 8 - 12 | 2 - 8 | 1 - 6 | 2 - 8 | |
| Average wax particle size (microns or µ) | 10 | 6 | 5 | 6 | |

| | | | | | |
|---|---|---|---|---|---|
| * particle size is based on microscopic images of the wax dispersions in Table I and showed that aqueous dispersions of solid wax particles having different particle size ranges or distribution were obtained, depending on the amounts and/or types of surfactants used | | | | | |

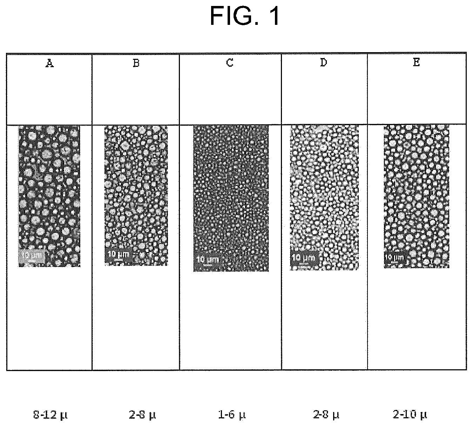

FIG. 1 shows the optical microscopic images of the exemplary beeswax dispersions in Table 1 above.

### Example II

**Table 2: Examples of aqueous dispersions containing phyto olive wax individually prepared according to the Wax Dispersion Protocol above**

| Ingredient | Aqueous Dispersions % weight of ingredient | | | | | | | | | | |
|---|---|---|---|---|---|---|---|---|---|---|---|
| | F | G | H | I | J | K | L | M | N | O | P |
| AA | 30 | 30 | 30 | 30 | 30 | 30 | 30 | 25 | 20 | 20 | 25 |
| BB | 2.7 | 2.5 | 2.3 | 2.1 | 1.3 | 1.1 | 2.5 | 2.5 | 2.0 | 1.0 | 2.5 |
| CC | - | - | - | - | - | - | 0.5 | 0.5 | 0.5 | 0.25 | 0.5 |
| DD | 0.3 | 0.5 | 0.7 | 0.9 | 0.2 | 0.4 | 0.5 | 0.5 | 0.5 | 0.25 | 0.5 |
| EE | 0.5 | 0.5 | 0.5 | 0.5 | 0.5 | 0.5 | 0.5 | 0.5 | 0.5 | 0.5 | 0.5 |
| FF | Q.S. to 100 | Q.S. to 100 | Q.S. to 100 | Q.S. to 100 | Q.S. to 100 | Q.S. to 100 | Q.S. to 100 | Q.S. to 100 | Q.S. to 100 | Q.S. to 100 | Q.S. to 100 |
| GG | 3-15 | 2-15 | 2-10 | 3-12 | 1-12 | 1-20 | 2-10 | 2-10 | 1-8 | 1-8 | 2-10 |
| HH | 10 | 10 | 8 | 10 | 10 | 15 | 8 | 8 | 6 | 6 | 8 |

| | | | | | | | | | | | |
|---|---|---|---|---|---|---|---|---|---|---|---|
| *speed of mixing to make the dispersion was at 3000 rpm for all dispersions | | | | | | | | | | | |

Details of the ingredients listed in above table:
- AA: = Phyto Olive wax (hydrogenated myristyl olive esters)
- BB: = PEG-30 glyceryl stearate (nonionic surfactant)
- CC: = Sorbitan palmitate(nonionic surfactant)
- DD: = Disodium stearoyl glutamate(anionic surfactant)
- EE: = phenoxyethanol (preservative)
- FF: = Deionized Water
- GG: = Solid wax Particle size range by image analysis (microns)
- HH: = Average wax particle size (microns)

Microscopic images of the aqueous dispersions in Table 2 showed that aqueous dispersions of solid wax particles having different particle size ranges or distribution were obtained, depending on the amounts of the surfactants and/or wax and on the types of surfactants used.

Aqueous dispersions whose surfactant solution consisted of only the nonionic surfactant, e.g., PEG-30 glyceryl stearate, or in combination with another nonionic surfactant, e.g., sorbitan palmitate, were found to be unstable, that is, the wax particles agglomerated in the absence of an ionic surfactant in the dispersion.

### Example III - Examples of formulations containing the aqueous dispersion

Formulas 1 and 2 below were individually prepared from an aqueous dispersion prepared according to the procedure described above and containing 30% beeswax, 2.7% of PEG-30 glyceryl stearate, 0.3% of disodium stearoyl glutamate and water (Q.S.) and adding an aliquot of the dispersion to the rest of the ingredients in Formula 1 or Formula 2.

Formula 3 below was prepared from an aqueous dispersion prepared according to the procedure described above and containing 25% silicone wax, 2.7% of cetyl PEG/PPG-10/1 dimethicone, 0.3% disodium stearoyl glutamate, and water (Q.S.). An aliquot of the dispersion was added to the rest of the ingredients in Formula 3.

Note that the particle sizes of the formulas presented below were measured by image analysis from microscopic images of the dispersions.

**Table 3: Gel-Lotion/Pump Spray Formula 1 (formulated as a gel-lotion or as a non-aerosol spray)**

| Ingredient | % weight |
|---|---|
| Beeswax Particles (average particle size 10 microns) | 5 |
| Polyacrylate-3 (23% active) | 2 |
| PEG-30 Glyceryl Stearate | 0.415 |
| Phenoxyethanol | 0.08 |
| Disodium Stearoyl Glutamate | 0.083 |
| Triethanolamine | 0.2 |
| Water | Q.S. to 100 |

**[000388]**

**Table 4: Mousse or (foam), Formula 2 (aerosolized)**

| Ingredient | % weight |
|---|---|
| Beeswax Particles (average particle size 10 microns) | 4.7 |
| Polyacrylate-3 (23% active) | 1.88 |
| PEG-30 Glyceryl Stearate | 0.390 |
| Phenoxyethanol | 0.0752 |
| Disodium Stearoyl Glutamate | 0.078 |
| Triethanolamine | 0.188 |
| A-70 propellant | 6 |
| Water | Q.S. to 100 |

**[000389]**

**Table 5: Gel Formula 3 (formulated as a gel-lotion or as a non-aerosol spray)**

| Ingredient | % weight |
|---|---|
| C30-45 ALKYLDIMETHYLSILYL POLYPROPYLSILSESQUIOXANE (silicone wax) (average particle size 15 microns) | 5.0 |
| CETYL PEG/PPG-10/1 DIMETHICONE | 0.6 |
| POLYACRYLATE-3 | 0.46 |
| TRIETHANOLAMINE | 0.2 |
| PHENOXYETHANOL | 0.08 |
| SODIUM LAURYL SULFATE | 0.02 |
| DISODIUM STEAROYL GLUTAMATE | 0.083 |
| PENTAERYTHRITYL TETRA-DI-T-BUTYL HYDROXYHYDROCINNAMATE | 0.00003 |
| PARAFFIN | 0.015 |
| Water | Q.S. to 100 |

### APPLICATION ONTO HAIR

The aqueous dispersion, C, in Table 1 and Formulas 1 and 2 in Tables 3 and 4 were applied onto hair in order to form a film or coating on the surface of hair.

It was observed that after treating the hair with the formulas and heating the treated hair using a blow dryer, the hair was easily configured into a desired shape. Upon cooling, it was observed that the formulas did not give a sticky or tacky feel to the hair. Moreover, upon re-heating the hair with the blow-dryer, the hair could be re-positioned/reshaped to a different configuration without having to reapply the formulas onto the hair.

On the other hand, Formulas 1 and 2 were tested on hair on the head of human subjects. After applying Formula 1 or Formula 2 with a comb or with the fingers onto dry or damp hair, the hair was heated using a blow drier after which the hair was styled/shaped as the temperature on the hair cooled down to room temperature. Upon cooling, it was observed that the formulas did not give a sticky or tacky feel to the hair. When more shaping and re-positioning of the hair was desired, the hair was heated again and the hair was re-positioned/re-shaped a different configuration without compromising the characteristics related to styling upon each application of heat the hair and without having to reapply the formulas onto the hair. Thus, it was possible to style the hair in several ways (e.g., helmet look, straightened curls, curling) using different curling/styling devices such as the flat iron or a comb without re-applying the formulas onto the hair. The formulas were easily removed from the hair with warm water and shampoo.

### Example IV

Based on the Wax Dispersion Protocol described above, aqueous wax dispersions were individually prepared/manufactured as follow:
A. Aqueous surfactant solution: the steps are the same as those described above in Example I.
B. Oil: Weighed amounts of wax (e.g., beeswax or phyto olive wax) and solvent (isododecane) were combined to give a total weight of 30 g; the resulting mixture were heated and melted as an oil phase for a few minutes in plastic covered beaker in a microwave (or other appropriate heating device).
C. Emulsification process
   1. While the aqueous surfactant solution was still at an elevated temperature (above room temperature, such as from 65°C to 70°C), the solution was mixed using a homogenizer/mixer (e.g., Silverson homogenizer) at a speed ranging from 3000 to 9000 rpm until bubbles were observed.
   2. The melted hot wax was added to the surfactant solution close to the mixing head of the homogenizer while mixing; the total weight of the resulting composition was 100 g.
   3. Once all the wax was added, mixing was continued for at least 5 minutes.
   4. The homogenizer blade was removed and the wax emulsion (dispersion) was mixed and cooled slightly towards room temperature before transferring into another container.
   5. The dispersion was stored at room temperature.
   6. The procedure above was followed for preparing other aqueous wax dispersions of the present invention using different waxes and/or surfactants at different levels.
   7. The particle sizes of the solid wax particles were measured using image analysis (microscopy) and/or laser diffraction methods and/or by particle size analyzer to obtain an average particle size. For example, particle size or particle size distribution can be measured by a Shimadzu SALD-7001 laser diffraction particle size analyzer, using quartz tubes having a refractive index of 1.2.

### Example IV: Table 6 - Examples of aqueous dispersions

| Ingredients | % by weight | | | | |
|---|---|---|---|---|---|
| PEG-30 glyceryl stearate HLB = 15 | 2.5% | 2.5% | 2.5% | 2.5% | 2.5% |
| Disodium stearoyl glutamate | 0.5% | 0.5% | 0.5% | 0.5% | 0.5% |
| Isododecane | 30% | 1% | 2% | 5% | 10% |
| Beeswax | 0% | 29% | 28% | 25% | 20% |
| DI Water w/ 0.5% Phenoxyethanol | 67% | 67% | 67% | 67% | 67% |
| Mixing at 3000 rpm 65 - 70 °C | | | | | |

### Example V: Testing the effect of varying levels of isododecane

Four wax dispersions were prepared; these dispersions contained beeswax at different amounts of 29, 28, 25 and 20 g and isododecane at different amounts of 1, 2, 5, and 10 g, respectively. The mixtures were evaluated with respect to how soft the wax dispersion material became with increasing isododecane concentration. As the isododecane level increased, the wax dispersion material became softer, an attribute which provides better spreadability and feel of the wax dispersion on the hair.

### Example VI

Based on the process described above, aqueous wax dispersions were individually prepared/manufactured as follow ("Wax Dispersion Protocol"):
A. Aqueous surfactant solution: the steps are the same as those described above in Example I.
B. Oil: Weighed amounts of the wax (e.g., beeswax or phyto olive wax) and a sunscreen agent (e.g., octocrylene or Mexoryl XL) were heated and melted for a few minutes in a microwave (or other appropriate heating device).
C. Emulsification process
   1. While the aqueous surfactant solution was still at an elevated temperature (above room temperature), the solution was mixed using a homogenizer/mixer (e.g., Silverson homogenizer) at a speed ranging from 3000 to 9000 rpm until bubbles were observed.
   2. The melted hot wax was added to the surfactant solution close to the mixing head of the homogenizer while mixing.
   3. Once all the wax was added, mixing was continued for at least 5 minutes.
   4. The homogenizer blade was removed and the wax emulsion (dispersion) was mixed and cooled slightly towards room temperature before transferring into another container.
   5. The dispersion was stored at room temperature.
   6. The procedure above was followed for preparing other aqueous wax dispersions of the present invention using different waxes and/or surfactants at different levels.
   7. The particle sizes of the solid wax particles were measured using image analysis (microscopy) and/or laser diffraction methods. For example, the particle size or particle size distribution was measured by a Shimadzu SALD-7001 laser diffraction particle size analyzer, using quartz tubes having a refractive index of 1.2.

### EXAMPLE VI - Table 7 - AQUEOUS WAX DISPERSIONSCONTAINING SUNSCREEN AGENTS

| | Dispersions % weight of ingredient | |
|---|---|---|
| Ingredient | A Octocrylene | B Mexoryl XL |
| Beeswax | 28 | 28 |
| Sunscreen Filter | 2 | 2 |
| PEG-30 glyceryl stearate (nonionic surfactant) | 2.5 | 2.5 |
| Disodium stearoyl glutamate (anionic surfactant) | 0.5 | 0.5 |
| phenoxyethanol (preservative) | 0.5 | 0.5 |
| Deionized Water | Q.S. to 100 | Q.S. to 100 |
| Speed of mixing | 3000 | 3000 |
| Solid wax particle size range (µm) | 2 - 12 | 2 - 20 |

### Example VIII: Formulations containing the aqueous dispersion

The aqueous dispersion can also be employed in a shampoo formulation as shown below.

Hair care (cleansing/conditioning) composition, Formula 4

| Ingredient | % weight |
|---|---|
| Beeswax Particles (average particle size 10 microns) | 5.0 |
| PEG-30 Glyceryl Stearate | 0.415 |
| Disodium Stearoyl Glutamate | 0.083 |
| Sodium laureth sulfate | 8.0 |
| Coco-betaine, Cocamide MIPA | 5.1 |
| Laureth-5 carboxylic acid | 0.7 |
| Silicone | 0.6 |
| Hexylene glycol, Propylene glycol | 0.9 |
| Polyquaternium-7 | 0.5 |
| PEG-55 propylene glycol oleate | 0.3 |
| PEG-60 Hydrogenated castor oil | 0.15 |
| preservatives, plant extracts, pH adjusters and Water | Q.S. to 100 |

The hair on the head of human subjects was shampooed with Formula 4 and rinsed. The hair was subsequently exposed to heat by blow drying. It was found that the hair could be shaped according to the desired style without having to apply any other styling or hair cosmetic composition onto the hair. It was also found that the formula provided a good curl hold/retention.

The following formulas demonstrate the use of other types of waxes comprising the solid wax particles of the aqueous dispersions of the present disclosure (formulas 5 to 7).

Hair styling composition containing phyto olive wax, Formula 5

| INCI Name | % by weight |
|---|---|
| POLYACRYLATE-3 | 0.46 |
| TRIETHANOLAMINE | 0.2 |
| PHENOXYETHANOL | 0.08 |
| SODIUM LAURYL SULFATE | 0.02 |
| HYDROGENATED MYRISTYL OLIVE ESTERS (phyto olive wax) (particle size range of 2 - 10 microns) | 5.0 |
| SORBITAN PALMITATE | 0.1 |
| DISODIUM STEAROYL GLUTAMATE | 0.1 |
| PEG-30 GLYCERYL STEARATE | 0.5 |
| WATER | Q.S. 100 |

Formula 5 above provided more shine and a sleek look to hair compared to a formula containing the beeswax aqueous dispersion (formula 1).

Hair styling composition containing ditrimethyloylpropane tetrastearate wax, Formula 6

| INCI Name | % by weight |
|---|---|
| POLYACRYLATE-3 | 0.46 |
| TRIETHANOLAMINE | 0.2 |
| PHENOXYETHANOL | 0.08 |
| SODIUM LAURYL SULFATE | 0.02 |
| DITRIMETHYLOLPROPANE TETRASTEARATE (particle size range of 5 - 20 microns) | 5.0 |
| DISODIUM STEAROYL GLUTAMATE | 0.083 |
| PEG-30 GLYCERYL STEARATE | 0.415 |
| WATER | Q.S. 100 |

Formula 6 above provided improved styleability to hair, a tighter curl hold, more shape memory, more body and a sleeker look to hair compared to a formula containing the beeswax aqueous dispersion (formula 1).

Hair styling composition containing VP/eicosene polymer wax, Formula 7

| INCI Name | % by weight |
|---|---|
| POLYACRYLATE-3 | 0.46 |
| TRIETHANOLAMINE | 0.2 |
| PHENOXYETHANOL | 0.08 |
| SODIUM LAURYL SULFATE | 0.02 |
| VP/EICOSENE COPOLYMER (particle size range of 2 - 8 microns) | 5.0 |
| DISODIUM STEAROYL GLUTAMATE | 0.083 |
| PEG-30 GLYCERYL STEARATE | 0.415 |
| WATER | Q.S. 100 |

Formula 7 above provided a tighter curl pattern to hair compared to a convention formula for curling or maintaining the curl of hair.

From the results observed from the use of the formulas above with respect to styling and shaping hair, it was found that different styling and shaping effects on the hair can be obtained, depending on the type of wax used in the aqueous dispersion.

### Example IX - Comparative Example

The effect of styling hair with a formula containing an aqueous dispersion of beeswax prepared according to the procedure described above was compared to that of styling hair with a conventional wax formula for styling hair. The conventional wax formula was prepared using traditional methods of preparing a wax hair styling product wherein the wax is not pre-dispersed and is directly added to rest of the ingredients.

Gel composition containing a beeswax aqueous dispersion, Formula 8

| INCI Name | % by weight |
|---|---|
| POLYACRYLATE-3 (in Viscophobe DB 1000) | 0.46 |
| TRIETHANOLAMINE | 0.2 |
| PHENOXYETHANOL | 0.08 |
| SODIUM LAURYL SULFATE | 0.02 |
| BEESWAX (particle size range of 8 - 10 microns) | 5.0 |
| SORBITAN PALMITATE | 0.1 |
| DISODIUM STEAROYL GLUTAMATE | 0.08 |
| PEG-30 GLYCERYL STEARATE | 0.4 |
| WATER | Q.S. 100 |

Conventional wax formula, Formula 9

| INCI Name | % by weight |
|---|---|
| BEESWAX | 10.0 |
| VA/VINYL BUTYL BENZOATE/CROTONATES COPOLYMER | 4.0 |
| ACRYLATES/C10-30 ALKYL ACRYLATE CROSSPOLYMER | 0.5 |
| TRIBEHENIN | 3.0 |
| NONIONIC SURFACTANTS (ALKOXYLATED FATTY ALCOHOLS) | 4.3 |
| FATTY ALCOHOL | 6.8 |
| GLYCERIN, PROPYLENE GLYCOL | 4 |
| CAPRYLIC/CAPRIC TRIGLYCERIDE, PEG-40 HYDROGENATED CASTOR OIL | 14.2 |
| PPG-2 MYRISTYL ETHER PROPIONATE, BIS-DIGLYCERYL POLYACYLADIPATE-2 | 7.4 |
| AMINOMETHYL PROPANOL | 0.92 |
| PRESERVATIVE, FRAGRANCE, SODIUM CHLORIDE, MAGNESIUM CHLORIDE, COLORANTS, HYDROLYZED WHEAT STARCH | 0.7 |
| WATER | Q.S. 100 |

When Formulas 8 and 9 were tested on the hair of human volunteers, formula 8 provided more root lift, volume, body and restyleability to the hair. Moreover, the hair dressers preferred formula 8 because it was less sticky on the hair, had a better feel on the hands, and provided better wettability to the hair.

## Claims

1. A composition comprising
- an aqueous dispersion comprising:
(a) at least one solid wax particle having a particle size ranging from equal to or greater than 1 micron to 25 microns and comprising at least one wax having a melting point of greater than 35°C, preferably ranging from 40°C to 100°C;
(b) a surfactant mixture comprising:
(i) at least one nonionic surfactant having an HLB of at least 5, and chosen from polyethylene glycol ethers of glyceryl esters, sorbitan esters, silicone-based emulsifying polymers having alkoxylated groups and/or side chains, and mixtures thereof; and
(ii) at least one ionic surfactant comprising at least one anionic surfactant chosen from alkyl sulfates and their salts, alkyl ether sulfates and their salts, acyl glutamates, alkyl ether carboxylates, and mixtures thereof; and
(c) water,
- and a carrier preferably being a cosmetically acceptable carrier chosen from water, volatile organic solvents, non-volatile organic solvents, silicones, polyols, glycols, glycol ethers, oils, and mixtures thereof,
wherein the at least one wax is present in an amount of from 1% to 5% by weight based on the total weight of the composition.

2. The composition of claim 1, wherein the least one wax is chosen from beeswax, hydrogenated myristyl olive esters, hydrogenated stearyl olive esters, VP/eicosene copolymer, ditrimethyloylpropane tetrastearate, and silsesquioxane resin wax, such as C30-45 alkyldimethylsilyl propylsilsesquioxane.

3. The composition of claim 1 or 2, wherein the at least one wax is present in an amount of from 10% to 80% by weight, preferably from 20% to 40% by weight, based on the total weight of the aqueous dispersion.

4. The composition of any one of the preceding claims, wherein the at least one wax has a hardness value of from 0.001 MPa to 15 MPa, preferably from 3 MPa to 10 MPa.

5. The composition of any one of the preceding claims, wherein (a) has a particle size of from 5 microns to 25 microns.

6. The composition of any one of the preceding claims, wherein (b)(i) is chosen from PEG-30 glyceryl stearate, sorbitan palmitate, Cetyl PEG/PPG-10/1 Dimethicone, Bis-PEG/PPG-16/16 PEG/PPG-16/16 Dimethicone, Bis-PEG/PPG-20/5 PEG/PPG-20/5 Dimethicone, PEG/PPG-25/4 Dimethicone, Bis-(Glyceryl/Lauryl) Glyceryl Lauryl Dimethicone, Bis-PEG/PPG-14/14 Dimethicone, and mixtures thereof.

7. The composition of any one of the preceding claims, wherein (b)(ii) comprises at least one anionic surfactant chosen from disodium stearoyl glutamate and sodium stearoyl glutamate.

8. The composition of any one of the preceding claims, wherein (b)(ii) comprises at least one cationic surfactant, preferably chosen from cetrimonium chloride, behentrimonium chloride, Dipalmitoylethyl hydroxyethylmonium methosulfate, Distearoylethyl hydroxyethylmonium methosulfate, and mixtures thereof.

9. The composition of any one of the preceding claims, wherein (b)(ii) is present in an amount of from 5% to 30% by weight, preferably from 5% to 20% by weight, based on the total weight of (b).

10. The composition of any one of the preceding claims, wherein (b) is present in an amount of from 1% to 3% by weight, preferably from 1.5% to 3% by weight, based on the total weight of the aqueous dispersion.

11. The composition of any one of the preceding claims, wherein (b) is free of amphoteric surfactants.

12. The composition of any one of the preceding claims, wherein the aqueous dispersion further comprises at least one sunscreen agent preferably chosen from octocrylene, drometrizole trisiloxane and mixtures, thereof, in particular in an amount from 0.1% to 6% by weight, based on the total weight of the aqueous dispersion.

13. The composition of any one of the preceding claims, wherein the aqueous dispersion further comprises at least one volatile solvent comprising preferably isododecane, in particular in an amount of from 1% to 10% by weight, based on the total weight of the aqueous dispersion.

14. The composition of any one of the preceding claims, wherein the at least one wax is present in an amount of from 2% to 5% by weight, based on the total weight of the composition.

15. Use of the composition of any one of the preceding claims, wherein the composition is heat-activated.

16. A method of coating hair, the method comprising:
(a) applying onto the hair, a composition according to any one of claims 1 to 14,
and
(b) heating the hair in order to melt the at least one solid wax particle.

17. A method of shaping hair comprising the method according to claim 16.

## Patentansprüche

1. Zusammensetzung, umfassend
- eine wässrige Dispersion, umfassend:
(a) mindestens ein festes Wachsteilchen, das eine Teilchengröße im Bereich von gleich oder größer als 1 Mikrometer bis 25 Mikrometer aufweist und mindestens ein Wachs umfasst, das einen Schmelzpunkt von über 35 °C, vorzugsweise im Bereich von 40 °C bis 100 °C aufweist;
(b) ein Tensidgemisch, umfassend:
(i) mindestens ein nichtionisches Tensid, das einen HLB-Wert von mindestens 5 aufweist und aus Polyethylenglycolethern von Glycerylestern, Sorbitanestern, auf Silikon basierenden emulgierenden Polymeren, die alkoxylierte Gruppen und/oder Seitenketten aufweisen, und Gemischen davon ausgewählt ist; und
(ii) mindestens ein ionisches Tensid, das mindestens ein anionisches Tensid umfasst, das aus Alkylsulfaten und ihren Salzen, Alkylethersulfaten und ihren Salzen, Acylglutamaten, Alkylethercarboxylaten und Gemischen davon ausgewählt ist; und
(c) Wasser,
- und einen Träger, der vorzugsweise ein kosmetisch annehmbarer Träger ist, der aus Wasser, flüchtigen organischen Lösungsmitteln, nicht flüchtigen organischen Lösungsmitteln, Silikonen, Polyolen, Glycolen, Glycolethern, Ölen und Gemischen davon ausgewählt ist, wobei das mindestens eine Wachs in einer Menge von 1 Gew.-% bis 5 Gew.-%, bezogen auf das Gesamtgewicht der Zusammensetzung, vorhanden ist.

2. Zusammensetzung nach Anspruch 1, wobei das mindestens eine Wachs aus Bienenwachs, hydrierten Myristylolivenestern, hydrierten Stearylolivenestern, VP/Eicosen-Copolymer, Ditrimethyloylpropantetrastearat und Silsesquioxanharzwachs wie C30-45-Alkyldimethylsilylpropylsilsesquioxan ausgewählt ist.

3. Zusammensetzung nach Anspruch 1 oder 2, wobei das mindestens eine Wachs in einer Menge von 10 Gew.-% bis 80 Gew.-%, vorzugsweise von 20 Gew.-% bis 40 Gew.-%, bezogen auf das Gesamtgewicht der wässrigen Dispersion, vorhanden ist.

4. Zusammensetzung nach einem der vorstehenden Ansprüche, wobei das mindestens eine Wachs einen Härtewert von 0,001 MPa bis 15 MPa, vorzugsweise von 3 MPa bis 10 MPa, aufweist.

5. Zusammensetzung nach einem der vorstehenden Ansprüche, wobei (a) eine Teilchengröße von 5 Mikrometer bis 25 Mikrometer aufweist.

6. Zusammensetzung nach einem der vorstehenden Ansprüche, wobei (b) (i) aus PEG-30-Glycerylstearat, Sorbitanpalmitat, Cetyl-PEG/PPG-10/1-Dimethicon, Bis-PEG/PPG-16/16 PEG/PPG-16/16-Dimethicon, Bis-PEG/PPG-20/5 PEG/PPG-20/5-Dimethicon, PEG/PPG-25/4-Dimethicon, Bis-(Glyceryl/Lauryl) Glyceryl-Lauryl-Dimethicon, Bis-PEG/PPG-14/14-Dimethicon und Gemischen davon ausgewählt ist.

7. Zusammensetzung nach einem der vorstehenden Ansprüche, wobei (b) (ii) mindestens ein anionisches Tensid umfasst, das aus Dinatriumstearoylglutamat und Natriumstearoylglutamat ausgewählt ist.

8. Zusammensetzung nach einem der vorstehenden Ansprüche, wobei (b)(ii) mindestens ein kationisches Tensid umfasst, das vorzugsweise aus Cetrimoniumchlorid, Behentrimoniumchlorid, Dipalmitoylethylhydroxyethylmoniummethosulfat, Distearoylethylhydroxyethylmoniummethosulfat und Gemischen davon ausgewählt ist.

9. Zusammensetzung nach einem der vorstehenden Ansprüche, wobei (b) (ii) in einer Menge von 5 Gew.-% bis 30 Gew.-%, vorzugsweise von 5 Gew.-% bis 20 Gew.-%, bezogen auf das Gesamtgewicht von (b), vorhanden ist.

10. Zusammensetzung nach einem der vorstehenden Ansprüche, wobei (b) in einer Menge von 1 Gew.-% bis 3 Gew.-%, vorzugsweise von 1,5 Gew.-% bis 3 Gew.-%, bezogen auf das Gesamtgewicht der wässrigen Dispersion, vorhanden ist.

11. Zusammensetzung nach einem der vorstehenden Ansprüche, wobei (b) frei von amphoteren Tensiden ist.

12. Zusammensetzung nach einem der vorstehenden Ansprüche, wobei die wässrige Dispersion ferner mindestens ein Sonnenschutzmittel, das vorzugsweise aus Octocrylen, Drometrizoltrisiloxan und Gemischen davon ausgewählt ist, insbesondere in einer Menge von 0,1 Gew.-% bis 6 Gew.-%, bezogen auf das Gesamtgewicht der wässrigen Dispersion, umfasst.

13. Zusammensetzung nach einem der vorstehenden Ansprüche, wobei die wässrige Dispersion ferner mindestens ein flüchtiges Lösungsmittel, das vorzugsweise Isododecan umfasst, insbesondere in einer Menge von 1 Gew.-% bis 10 Gew.-%, bezogen auf das Gesamtgewicht der wässrigen Dispersion, umfasst.

14. Zusammensetzung nach einem der vorstehenden Ansprüche, wobei das mindestens eine Wachs in einer Menge von 2 Gew.-% bis 5 Gew.-%, bezogen auf das Gesamtgesicht der Zusammensetzung, vorhanden ist.

15. Verwendung der Zusammensetzung nach einem der vorstehenden Ansprüche, wobei die Zusammensetzung hitzeaktiviert ist.

16. Verfahren zum Beschichten von Haar, wobei das Verfahren Folgendes umfasst:
(a) Aufbringen einer Zusammensetzung nach einem der Ansprüche 1 bis 14 auf das Haar und
(b) Erhitzen des Haars, um das mindestens eine feste Wachsteilchen aufzuschmelzen.

17. Verfahren zum Formen von Haar, umfassend das Verfahren nach Anspruch 16.

## Revendications

1. Composition comprenant
- une dispersion aqueuse comprenant :
(a) au moins une particule de cire solide ayant une taille de particule dans la plage de 1 micron ou plus à 25 microns et comprenant au moins une cire possédant un point de fusion supérieur à 35 °C, préférablement dans la plage de 40 °C à 100 °C ;
(b) un mélange de tensioactifs comprenant :
(i) au moins un tensioactif non ionique possédant une HLB d'au moins 5, et choisi parmi des éthers de polyéthylène glycol d'esters de glycéryle, des esters de sorbitane, des polymères émulsifiants à base de silicone possédant des groupes alcoxylés et/ou des chaînes latérales, et leurs mélanges; et
(ii) au moins un tensioactif ionique comprenant au moins un tensioactif anionique choisi parmi des alkylsulfates et leurs sels, des alkyléthersulfates et leurs sels, des acylglutamates, des alkyléthercarboxylates, et leurs mélanges; et
(c) de l'eau,
- et un support qui est préférablement un support acceptable sur le plan cosmétique choisi parmi l'eau, des solvants organiques volatils, des solvants organiques non volatils, des silicones, des polyols, des glycols, des éthers de glycol, des huiles, et leurs mélanges,
dans laquelle la cire, au nombre d'au moins une, est présente en une quantité allant de 1 % à 5 % en poids sur la base du poids total de la composition.

2. Composition selon la revendication 1, dans laquelle la cire, au nombre d'au moins une, est choisie parmi la cire d'abeille, des esters de myristyle et d'huile d'olive hydrogénée, des esters de stéaryle et d'huile d'olive hydrogénée, un copolymère de VP/éicosène, le tétrastéarate de ditriméthyloylpropane et une cire de résine de silsesquioxane, tel qu'un (C30-45 alkyl)diméthylsilyl-propylsilsesquioxane.

3. Composition selon la revendication 1 ou 2, dans laquelle la cire, au nombre d'au moins une, est présente en une quantité allant de 10 % à 80 % en poids, préférablement de 20 % à 40 % en poids, sur la base du poids total de la dispersion aqueuse.

4. Composition selon l'une quelconque des revendications précédentes, dans laquelle la cire, au nombre d'au moins une, présente une valeur de dureté allant de 0,001 MPa à 15 MPa, préférablement de 3 MPa à 10 MPa.

5. Composition selon l'une quelconque des revendications précédentes, dans laquelle (a) a une taille de particule allant de 5 microns à 25 microns.

6. Composition selon l'une quelconque des revendications précédentes, dans laquelle (b)(i) est choisi parmi le stéarate de glycéryle PEG-30, le palmitate de sorbitane, la cétyl-PEG/PPG-10/1 diméthicone, la Bis-PEG/PPG-16/16 PEG/PPG-16/16 diméthicone, la Bis-PEG/PPG-20/5 PEG/PPG-20/5 diméthicone, la PEG/PPG-25/4 diméthicone, la Bis-(glycéryl/lauryl) glycéryl-lauryl-diméthicone, la Bis-PEG/PPG-14/14 diméthicone, et leurs mélanges.

7. Composition selon l'une quelconque des revendications précédentes, dans laquelle (b)(ii) comprend au moins un tensioactif anionique choisi parmi le stéaroylglutamate disodique et le stéaroylglutamate de sodium.

8. Composition selon l'une quelconque des revendications précédentes, dans laquelle (b)(ii) comprend au moins un tensioactif cationique, préférablement choisi parmi le chlorure de cétrimonium, le chlorure de béhentrimonium, le méthosulfate de dipalmitoyléthyl-hydroxyéthylmonium, le méthosulfate de distéaroyléthyl-hydroxyéthylmonium, et leurs mélanges.

9. Composition selon l'une quelconque des revendications précédentes, dans laquelle (b)(ii) est présent en une quantité allant de 5 % à 30 % en poids, préférablement de 5 % à 20 % en poids, sur la base du poids total de (b).

10. Composition selon l'une quelconque des revendications précédentes, dans laquelle (b) est présent en une quantité allant de 1 % à 3 % en poids, préférablement de 1,5 % à 3 % en poids, sur la base du poids total de la dispersion aqueuse.

11. Composition selon l'une quelconque des revendications précédentes, dans laquelle (b) est exempt de tensioactifs amphotères.

12. Composition selon l'une quelconque des revendications précédentes, dans laquelle la dispersion aqueuse comprend en outre au moins un agent de protection solaire préférablement choisi parmi l'octocrylène, le drométrizole trisiloxane et leurs mélanges, en particulier en une quantité de 0,1 % à 6 % en poids, sur la base du poids total de la dispersion aqueuse.

13. Composition selon l'une quelconque des revendications précédentes, dans laquelle la dispersion aqueuse comprend en outre au moins un solvant volatil comprenant préférablement l'isododécane, en particulier en une quantité allant de 1 % à 10 % en poids, sur la base du poids total de la dispersion aqueuse.

14. Composition selon l'une quelconque des revendications précédentes, dans laquelle la cire, au nombre d'au moins une, est présente en une quantité allant de 2 % à 5 % en poids, sur la base du poids total de la composition.

15. Utilisation de la composition selon l'une quelconque des revendications précédentes, dans laquelle la composition est activée thermiquement.

16. Procédé de revêtement de cheveux, le procédé comprenant :
(a) l'application sur les cheveux, d'une composition selon l'une quelconque des revendication 1 à 14, et
(b) le chauffage des cheveux afin de faire fondre la particule de cire solide au nombre d'au moins une.

17. Procédé de mise en forme des cheveux comprenant le procédé selon la revendication 16.
